# EUROPEAN PATENT SPECIFICATION

(11) **EP 3 096 759 B1**
(45) Date of publication and mention of the grant of the patent: **06.04.2022**
(21) Application number: 15740303.1
(22) Date of filing: 21.01.2015
(51) Int. Cl.: A61K 31/451, C07D 211/20, C07D 211/24, A61K 9/16, A61P 25/08, A61P 25/16, A61P 25/28

(54) **MODIFIED RELEASE FORMULATIONS OF PRIDOPIDINE**
FORMULIERUNGEN AUS PRIDOPIDIN MIT MODIFIZIERTER FREISETZUNG
FORMULATIONS À LIBÉRATION MODIFIÉE DE PRIDOPIDINE

(30) Priority: 22.01.2014 US 201461930358 P; 15.09.2014 US 201462050626 P
(43) Date of publication of application: 30.11.2016
(73) Proprietor: Prilenia Neurotherapeutics Ltd., Herzliya 4672561 (IL)
(72) Inventor: LICHT, Daniella, Givat Shmuel (IL); LOVINGER, Ioana, 4424541 Kfar Saba (IL); GUILATT, Laura Yehudit, 60920 Kadima (IL); BASSAN, Merav, Ramat Gan 5253179 (IL)
(74) Representative: Pearl Cohen Zedek Latzer Baratz UK LLP
(86) International application number: PCT/US2015/012248
(87) International publication number: WO 2015/112601

(56) References cited:
- WO-A1-2014/205229
- US-A1- 2011 206 782
- US-A1- 2013 150 406
- US-A1- 2013 267 552
- MARTIN MICHL ET AL: "Pridopidine in the pharmacological treatment of Huntington?s disease", CLINICAL INVESTIGATION, vol. 3, no. 7, 1 July 2013 (2013-07-01), pages 691-699, XP055382520, UK ISSN: 2041-6792, DOI: 10.4155/cli.13.54

## Description

Throughout this application, various publications are referred by first author and year of publication. Full citations for these publications are presented in a section entitled References immediately preceding the claims.

### BACKGROUND OF THE INVENTION

Pridopidine (Huntexil) is a unique compound developed for the treatment of patients with motor symptoms associated with Huntington's disease. Its chemical name is 4-(3-(Methylsulfonyl)phenyl)-1-propylpiperidine, and its Chemical Registry number is 882737-42-0 (U.S. Publication No. US-2013-0267552-A1). Processes of synthesis of pridopidine and a pharmaceutically acceptable salt thereof are disclosed in U.S. Patent No. 7,923,459. U.S. Patent No. 6,903,120 claims Pridopidine for the treatment of Parkinson's disease, dyskinesias, dystonias, Tourette's disease, iatrogenic and non-iatrogenic psychoses and hallucinoses, mood and anxiety disorders, sleep disorder, autism spectrum disorder, ADHD, Huntington's disease, age-related cognitive impairment, and disorders related to alcohol abuse and narcotic substance abuse.

### BRIEF SUMMARY OF THE INVENTION

This invention provides a modified release solid oral dosage form comprising a therapeutically effective amount of Pridopidine or a pharmaceutically acceptable salt thereof, and at least one pharmaceutically acceptable rate controlling excipient, wherein the rate controlling excipient is a hydrophilic or hydrophobic polymer selected from hydrogenated castor oil, polyethylene oxide, ethyl cellulose, hydroxypropyl methylcellulose (HPMC), hydroxypropyl cellulose (HPC), polyvinyl alcohol (PVA), vinyl alcohol polymer, polyacrylates, polymethacrylates, ethyl acrylate-methyl methacrylate copolymers, glyceryl monostearate, and mixtures thereof, wherein the solid oral dosage form provides an *in vivo* plasma pridopidine concentration profile having a Mean Cₘₐₓ of 1,400 ng/ml or less.

This invention also provides a modified release solid oral dosage form comprising a therapeutically effective amount of Pridopidine or a pharmaceutically acceptable salt thereof, and at least one pharmaceutically acceptable rate controlling excipient, wherein the rate controlling excipient is a hydrophilic or hydrophobic polymer selected from hydrogenated castor oil, polyethylene oxide, ethyl cellulose, hydroxypropyl methylcellulose (HPMC), hydroxypropyl cellulose (HPC), polyvinyl alcohol (PVA), vinyl alcohol polymer, polyacrylates, polymethacrylates, ethyl acrylate-methyl methacrylate copolymers, glyceryl monostearate, and mixtures thereof, and wherein the solid oral dosage form provides an *in vivo* plasma pridopidine concentration profile having a Cₘₐₓ from 244 ng/ml to 1568 ng/ml when given at steady state.

In an embodiment, the rate controlling excipient is a combination of at least a hydroxypropyl methylcellulose (HPMC) and hydrogenated castor oil.

In an embodiment, the modified release solid oral dosage form provides an *in vivo* plasma pridopidine concentration profile having a Cₘₐₓ from 244 ng/ml to 1002 ng/ml when given as single dose.

In an embodiment, the modified release solid oral dosage form provides an *in vivo* plasma pridopidine concentration profile having a Mean Cₘₐₓ and Mean AUCₜₐᵤ which is lower than a Mean Cₘₐₓ and Mean AUCₜₐᵤ respectively resulting from the b.i.d. administration of an immediate release solid oral dosage form which contains half the amount of the Pridopidine or a pharmaceutically acceptable salt thereof.

In an embodiment, the mean time required to reach the maximal plasma, serum or blood concentration of the drug, following administration of the drug is more than 2 hours.

In an embodiment, the total amount of the rate controlling excipients is from 8% to 70% of the total weight of the dosage form, from 10% to 50% of the total weight of the dosage form, or from 20% to 50% of the total weight of the dosage form, from 30% to 50% or from 30% to 40% of the total weight of the dosage form.

In an embodiment, the dosage form comprises between 22.5mg to 350mg pridopidine or a pharmaceutically acceptable salt thereof.

In an embodiment, the pridopidine or a pharmaceutically acceptable salt thereof comprises from 15% to 60% by weight of the dosage form.

In an embodiment, the weight ratio of pridopidine or the pharmaceutically acceptable salt thereof to the rate controlling excipient is from 0.2:1 to 1:1, preferably from 0.3:1 to 0.8:1, more preferably 0.5:1 to 0.7:1.

In an embodiment, the modified release solid oral dosage form further comprises ethylcellulose, wherein the total amount of the ethylcellulose is from 0.5% to 10% of the total weight of the dosage form.

In an embodiment, the modified release solid oral dosage form further comprises a mucoadhesive, wherein the mucoadhesive is selected from water soluble or water insoluble hydrophilic polymers, polymers that have swellable networks, hydrogels, and polymers with groups that can cross-link with other polymers or with a mucous membrane, wherein preferably the mucoadhesive is polyethylene oxide.

In an embodiment, the pharmaceutically acceptable salt of pridopidine is hydrochloride salt.

In an embodiment, the oral dosage form comprises capsules, tablets, pellets, granules, powders coated or uncoated and combination thereof.

The present invention also provides a pharmaceutical formulation comprising the modified release solid oral dosage form, and one or more pharmaceutically acceptable carriers or excipients selected from binders, fillers, plasticizers, glidants and lubricants and mixtures thereof.

In an embodiment, the binder is starch, pregeletinized starch, polyethylene oxide, cellulose polymers, hydroxypropylmethyl cellulose, hydroxypropylcellulose, methylcellulose, hydroxyethyl cellulose, polyvinylpyrrolidone, polyvinyl alcohol, or a mixture thereof.

In an embodiment, the filler is microcrystalline cellulose, sugar spheres, lactose, sorbitol, dextrose, sucrose, mannitol, dibasic or tribasic calcium phosphate, calcium sulfate, starch, retalac or a mixture thereof, optionally wherein the filler is a silicified microcrystalline cellulose.

In an embodiment, the plasticizer is polyethylene glycol, triethyl citrate, tributyl citrate, glycerin, dibutyl sebacate, triacetin, diethylphthalat or a mixture thereof.

In an embodiment, the glidant is starch, pregelatinized starch, silicone dioxide, colloidal silicone dioxide, talc or a mixture thereof.

In an embodiment, the lubricant is sodium stearyl fumarate, stearic acid, magnesium stearate, calcium stearate, zinc stearate, talc, glyceryl behenate, glyceryl monostearate or a mixture thereof.

The present invention also provides the modified release solid oral dosage form or pharmaceutical formulation for use in the treatment of Huntington's Disease, Parkinson's disease, iatrogenic and non-iatrogenic Parkinsonism, dyskinesias, dystonias, Tourette's disease, iatrogenic and non-iatrogenic psychoses and hallucinoses, schizophrenia disorder or schizophreniform disorder, mood and anxiety disorders, manodepressive illness, depression, obsessive-compulsive disease, a sleep disorder, autism spectrum disorder, ADHD, age-related cognitive impairment, abuse of alcohol and substances used as narcotics, Alzheimer's disease or Retts syndrome, wherein the modified release solid oral dosage form or pharmaceutical formulation is adapted for once daily administration.

### BRIEF DESCRIPTION OF THE SEVERAL VIEWS OF THE DRAWINGS

- *Figure 1:*: Pridopidine geometric mean plasma concentrations versus time from Example 1.
- *Figure 2:*: Observed and predicted relation between pridopidine plasma levels and ΔΔQTcF; the line represents population mean predictions.
- *Figure 3:*: In vitro dissolution rates of the dosage forms MR-1, MR-2 and MR-3.
- *Figure 4:*: *Plasma concentration-time profiles of pridopidine after single dose b.i.d. administration*: GastroPlus Method validation: Simulation single dose 22 mg IR pridopidine, and comparison to data from study. Figure 4a is simulated data and Figure 4b is data from study.
- *Figure 5:*: *Plasma concentration-time profiles of pridopidine after multiple dose b.i.d. administration*: GastroPlus Method validation: Simulation of (steady state) pharmacokinetic (PK) profile following 45 mg bid IR pridopidine, and comparison to data from study. Figure 5a is simulated data and Figure 5b is data from study.
- *Figure 6:*: (a-b) Mean plasma level curves of pridopidine after oral administration of pridopidine as various MR and reference IR formulations, 0-12h period (a) and semi-logarithmic presentation (b).

### DETAILED DESCRIPTION OF THE INVENTION

This invention provides a modified release solid oral dosage form comprising a therapeutically effective amount of Pridopidine or a pharmaceutically acceptable salt thereof, and at least one pharmaceutically acceptable rate controlling excipient, wherein the rate controlling excipient is a hydrophilic or hydrophobic polymer selected from hydrogenated castor oil, polyethylene oxide, ethyl cellulose, hydroxypropyl methylcellulose (HPMC), hydroxypropyl cellulose (HPC), polyvinyl alcohol (PVA), vinyl alcohol polymer, polyacrylates, polymethacrylates, ethyl acrylate-methyl methacrylate copolymers, glyceryl monostearate, and mixtures thereof, wherein the solid oral dosage form provides an *in vivo* plasma pridopidine concentration profile having a Mean Cₘₐₓ of about 1,400 ng/ml or less or having a Cₘₐₓ from about 244 ng/ml to about 1568 ng/ml at steady state.

In an embodiment, the solid oral dosage form provides an *in vivo* plasma pridopidine concentration profile having a Mean Cₘₐₓ of about 1,157 ng/ml or less.

In an embodiment, the solid oral dosage form provides an *in vivo* plasma pridopidine concentration profile having a Mean Cₘₐₓ of about 906 ng/ml or less.

In an embodiment, the solid oral dosage form provides an *in vivo* plasma pridopidine concentration profile having a Mean Cₘₐₓ of about 499 ng/ml or less.

In an embodiment, the solid oral dosage form provides an *in vivo* plasma pridopidine concentration profile having a Mean Cₘₐₓ of about 718 ng/ml or less measured after single dose administration.

In an embodiment, the solid oral dosage form provides an *in vivo* plasma pridopidine concentration profile having a Mean Cₘₐₓ of about 486 ng/ml or less measured after single dose administration.

In an embodiment, the solid oral dosage form provides an *in vivo* plasma pridopidine concentration profile having a Mean Cₘₐₓ of about 327 ng/ml or less measured after single dose administration.

In an embodiment, the solid oral dosage form provides an *in vivo* plasma pridopidine concentration profile having a Cₘₐₓ from about 382 ng/ml to about 1,568 ng/ml.

In an embodiment, the solid oral dosage form provides an *in vivo* plasma pridopidine concentration profile having a Cₘₐₓ from about 244 ng/ml to about 1,002 ng/ml. In another embodiment, the solid oral dosage form provides an *in vivo* plasma profile having a Cₘₐₓ between 244 ng/ml and 813 ng/ml. In another embodiment, the solid oral dosage form provides an *in vivo* plasma profile having a Cₘₐₓ between 493 ng/ml and 1,002 ng/ml. In an embodiment, the solid oral dosage form provides an *in vivo* plasma profile having a Cₘₐₓ between 324 ng/ml and 813 ng/ml. In an embodiment, the solid oral dosage form provides an *in vivo* plasma profile having a Cₘₐₓ between 871 ng/ml and 1,568 ng/ml.

In an embodiment, the solid oral dosage form provides an *in vivo* plasma profile having a Cₘₐₓ between 382 ng/ml and 1,287 ng/ml.

In an embodiment, the solid oral dosage form provides an *in vivo* plasma profile having a Cₘₐₓ between 639 ng/ml and 1,287 ng/ml.

In an embodiment, the Mean AUCₜₐᵤ is about 5,253 ng h/ml or more. In another embodiment, the Mean AUCₜₐᵤ is about 7,178 ng h/ml or more. In another embodiment, the Mean AUCₜₐᵤ is about 14,185 ng h/ml or more. In another embodiment, the Mean AUCₜₐᵤ is about 18,065 ng h/ml or more.

In an embodiment, the AUC_{0-inf} is about 2,249 ng h/ml or more. In another embodiment, the Mean AUC_{0-inf} is about 5,043 ng h/ml or more. In another embodiment, the Mean AUC_{0-inf} is about 7,897 ng h/ml or more. In another embodiment, the Mean AUC_{0-inf} is about 13,594 ng h/ml or more.

In an embodiment, the dosage form comprises from about 22.5mg to about 350mg Pridopidine or a pharmaceutically acceptable salt thereof. In another embodiment, the dosage form comprises from about 45mg to about 300mg Pridopidine or a pharmaceutically acceptable salt thereof.

In another embodiment, the dosage form comprises from about 90 to about 250mg Pridopidine or a pharmaceutically acceptable salt thereof. In another embodiment, the dosage form comprises at least about 90mg Pridopidine or a pharmaceutically acceptable salt thereof. In another embodiment, the dosage form comprises at least about 100mg Pridopidine or a pharmaceutically acceptable salt thereof. In another embodiment, the dosage form comprises at least about 125mg Pridopidine or a pharmaceutically acceptable salt thereof. In another embodiment, the dosage form comprises at least about 135mg Pridopidine or a pharmaceutically acceptable salt thereof. In another embodiment, the dosage form comprises at least about 150mg Pridopidine or a pharmaceutically acceptable salt thereof. In another embodiment, the dosage form comprises at least about 180mg Pridopidine or a pharmaceutically acceptable salt thereof or more. In another embodiment, the dosage form comprises at least about 200mg Pridopidine or a pharmaceutically acceptable salt thereof or more. In another embodiment, the dosage form comprises at least about 225mg Pridopidine or a pharmaceutically acceptable salt thereof or more. In an embodiment, the dosage form comprises at least about 250mg Pridopidine or a pharmaceutically acceptable salt thereof or more. In another embodiment, the dosage form comprises at least about 315mg Pridopidine or a pharmaceutically acceptable salt thereof or more. In another embodiment, the dosage form comprises about 90mg Pridopidine or a pharmaceutically acceptable salt thereof. In another embodiment, the dosage form comprises about 100mg Pridopidine or a pharmaceutically acceptable salt thereof. In another embodiment, the dosage form comprises about 125mg Pridopidine or a pharmaceutically acceptable salt thereof. In another embodiment, the dosage form comprises about 135mg Pridopidine or a pharmaceutically acceptable salt thereof. In another embodiment, the dosage form comprises about 150mg Pridopidine or a pharmaceutically acceptable salt thereof.

In another embodiment, the dosage form comprises about 180mg Pridopidine or a pharmaceutically acceptable salt thereof. In another embodiment, the dosage form comprises about 200mg Pridopidine or a pharmaceutically acceptable salt thereof. In another embodiment, the dosage form comprises about 225mg Pridopidine or a pharmaceutically acceptable salt thereof. In another embodiment, the dosage form comprises about 250mg Pridopidine or a pharmaceutically acceptable salt thereof. In another embodiment, the dosage form comprises about 315mg Pridopidine or a pharmaceutically acceptable salt thereof.

In an embodiment, the *in vivo* plasma profile is measured at steady state.

In an embodiment, the *in vivo* plasma profile is measured after single dose administration.

This invention also provides a modified release solid oral dosage form comprising a therapeutically effective amount of Pridopidine or a pharmaceutically acceptable salt thereof, and at least one pharmaceutically acceptable rate controlling excipient, and wherein the solid oral dosage form provides an *in vivo* plasma pridopidine concentration profile having a Mean Cₘₐₓ which is lower than a Mean Cₘₐₓ resulting from the b.i.d. administration of an immediate release solid oral dosage form which contains half the amount of the Pridopidine or a pharmaceutically acceptable salt thereof.

In an embodiment, the amount of Pridopidine or a pharmaceutically acceptable salt thereof is more than 45 mg of Pridopidine. In another embodiment, the amount of Pridopidine or a pharmaceutically acceptable salt thereof is at least about 90 mg of Pridopidine and the immediate release dosage form contains about 45 mg of Pridopidine. In another embodiment, the amount of Pridopidine or a pharmaceutically acceptable salt thereof is at least about 100 mg of Pridopidine and the immediate release solid oral dosage form contains about 45 mg of Pridopidine. In another embodiment, the amount of Pridopidine or a pharmaceutically acceptable salt thereof is at least about 125 mg of Pridopidine and the immediate release solid oral dosage form contains about 45 mg of Pridopidine. In another embodiment, the amount of Pridopidine or a pharmaceutically acceptable salt thereof is at least about 135 mg of Pridopidine and the immediate release solid oral dosage form contains about 45 mg of Pridopidine. In another embodiment, the amount of Pridopidine or a pharmaceutically acceptable salt thereof is at least about 135 mg of Pridopidine and the immediate release solid oral dosage form contains about 67.5 mg of Pridopidine. In another embodiment, the amount of Pridopidine or a pharmaceutically acceptable salt thereof is at least about 150 mg of Pridopidine and the immediate release solid oral dosage form contains about 45 mg of Pridopidine. In another embodiment, the amount of Pridopidine or a pharmaceutically acceptable salt thereof is at least about 150 mg of Pridopidine and the immediate release solid oral dosage form contains about 67.5 mg of Pridopidine. In another embodiment, the amount of Pridopidine or a pharmaceutically acceptable salt thereof is at least about 180 mg of Pridopidine and the immediate release solid oral dosage form contains about 45 mg of Pridopidine.

In another embodiment, the amount of Pridopidine or a pharmaceutically acceptable salt thereof is at least about 180 mg of Pridopidine and the immediate release solid oral dosage form contains about 67.5 mg of Pridopidine. In another embodiment, the amount of Pridopidine or a pharmaceutically acceptable salt thereof is at least about 180 mg of Pridopidine and the immediate release solid oral dosage form contains about 90 mg of Pridopidine. In another embodiment, the amount of Pridopidine or a pharmaceutically acceptable salt thereof is at least about 200 mg of Pridopidine and the immediate release solid oral dosage form contains about 45 mg of Pridopidine. In another embodiment, the amount of Pridopidine or a pharmaceutically acceptable salt thereof is at least about 200 mg of Pridopidine and the immediate release solid oral dosage form contains about 67.5 mg of Pridopidine. In another embodiment, the amount of Pridopidine or a pharmaceutically acceptable salt thereof is at least about 200 mg of Pridopidine and the immediate release solid oral dosage form contains about 90 mg of Pridopidine. In another embodiment, the amount of Pridopidine or a pharmaceutically acceptable salt thereof is at least about 225 mg of Pridopidine and the immediate release solid oral dosage form contains about 45 mg of Pridopidine. In another embodiment, the amount of Pridopidine or a pharmaceutically acceptable salt thereof is at least about 225 mg of Pridopidine and the immediate release solid oral dosage form contains about 67.5 mg of Pridopidine.

In another embodiment, the amount of Pridopidine or a pharmaceutically acceptable salt thereof is at least about 225 mg of Pridopidine and the immediate release solid oral dosage form contains about 90 mg of Pridopidine. In another embodiment, the amount of Pridopidine or a pharmaceutically acceptable salt thereof is at least about 225 mg of Pridopidine and the immediate release solid oral dosage form contains about 112.5 mg of Pridopidine. In another embodiment, the amount of Pridopidine or a pharmaceutically acceptable salt thereof is at least about 250 mg of Pridopidine and the immediate release solid oral dosage form contains about 45 mg of Pridopidine. In another embodiment, the amount of Pridopidine or a pharmaceutically acceptable salt thereof is at least about 250 mg of Pridopidine and the immediate release solid oral dosage form contains about 67.5 mg of Pridopidine. In another embodiment, the amount of Pridopidine or a pharmaceutically acceptable salt thereof is at least about 250 mg of Pridopidine and the immediate release solid oral dosage form contains about 90 mg of Pridopidine. In another embodiment, the amount of Pridopidine or a pharmaceutically acceptable salt thereof is at least about 250 mg of Pridopidine and the immediate release solid oral dosage form contains about 112.5 mg of Pridopidine. In another embodiment, the amount of Pridopidine or a pharmaceutically acceptable salt thereof is at least about 315 mg of Pridopidine and the immediate release solid oral dosage form contains about 45 mg of Pridopidine. In another embodiment, the amount of Pridopidine or a pharmaceutically acceptable salt thereof is at least about 315 mg of Pridopidine and the immediate release solid oral dosage form contains about 67.5 mg of Pridopidine. In another embodiment, the amount of Pridopidine or a pharmaceutically acceptable salt thereof is at least about 315 mg of Pridopidine and the immediate release solid oral dosage form contains about 90 mg of Pridopidine. In another embodiment, the amount of Pridopidine or a pharmaceutically acceptable salt thereof is at least about 315 mg of Pridopidine and the immediate release solid oral dosage form contains about 112.5 mg of Pridopidine. In another embodiment, the amount of Pridopidine or a pharmaceutically acceptable salt thereof is at least about 315 mg of Pridopidine and the immediate release solid oral dosage form contains about 157.5 mg of Pridopidine

In another embodiment, the amount of Pridopidine or a pharmaceutically acceptable salt thereof is about 90 mg. In another embodiment, the amount of Pridopidine or a pharmaceutically acceptable salt thereof is about 100 mg. In another embodiment, the amount of Pridopidine or a pharmaceutically acceptable salt thereof is about 125 mg. In another embodiment, the amount of Pridopidine or a pharmaceutically acceptable salt thereof is about 135 mg. In another embodiment, the amount of Pridopidine or a pharmaceutically acceptable salt thereof is about 150 mg. In another embodiment, the amount of Pridopidine or a pharmaceutically acceptable salt thereof is about 180 mg. In another embodiment, the amount of Pridopidine or a pharmaceutically acceptable salt thereof is about 200 mg. In another embodiment, the amount of Pridopidine or a pharmaceutically acceptable salt thereof is about 225 mg. In another embodiment, the amount of Pridopidine or a pharmaceutically acceptable salt thereof is about 250 mg. In another embodiment, the amount of Pridopidine or a pharmaceutically acceptable salt thereof is about 315 mg.

In an embodiment, the solid oral dosage form provides an *in vivo* plasma pridopidine concentration profile having a Mean AUCₜₐᵤ which is at least about 50% of the Mean AUCₜₐᵤ provided by the b.i.d. administration of an immediate release solid oral dosage form which contains half the amount of the Pridopidine or a pharmaceutically acceptable salt thereof. In another embodiment, the solid oral dosage form provides an *in vivo* plasma pridopidine concentration profile having a Mean AUCₜₐᵤ which is at least about 60% of the Mean AUCₜₐᵤ provided by the b.i.d. administration of an immediate release solid oral dosage form which contains half the amount of the Pridopidine or a pharmaceutically acceptable salt thereof. In another embodiment, the solid oral dosage form provides an *in vivo* plasma pridopidine concentration profile having a Mean AUCₜₐᵤ which is at least about 70% of the Mean AUCₜₐᵤ provided by the b.i.d. administration of an immediate release solid oral dosage form which contains half the amount of the Pridopidine or a pharmaceutically acceptable salt thereof. In another embodiment, the solid oral dosage form provides an *in vivo* plasma pridopidine concentration profile having a Mean AUCₜₐᵤ which is at least about 80% of the Mean AUCₜₐᵤ provided by the b.i.d. administration of an immediate release solid oral dosage form which contains half the amount of the Pridopidine or a pharmaceutically acceptable salt thereof. In another embodiment, the solid oral dosage form provides an *in vivo* plasma pridopidine concentration profile having a Mean AUCₜₐᵤ which is at least about 90% of the Mean AUCₜₐᵤ provided by the b.i.d. administration of an immediate release solid oral dosage form which contains half the amount of the Pridopidine or a pharmaceutically acceptable salt thereof. In another embodiment, the solid oral dosage form provides an *in vivo* plasma pridopidine concentration profile having a Mean AUCₜₐᵤ which is at least about 95% of the Mean AUCₜₐᵤ provided by the b.i.d. administration of an immediate release solid oral dosage form which contains half the amount of the Pridopidine or a pharmaceutically acceptable salt thereof.

In an embodiment, the b.i.d. administration of an immediate release solid oral dosage form has a time interval between doses of 5-10 hours. In another embodiment, the b.i.d. administration of an immediate release solid oral dosage form has a time interval between doses of 6-8 hours. In another embodiment, the b.i.d. administration of an immediate release solid oral dosage form has a time interval between doses of 6.5 hours. In another embodiment, the b.i.d. administration of an immediate release solid oral dosage form has a time interval between doses of 7 hours.

In an embodiment, the solid oral dosage form provides an *in vivo* plasma pridopidine concentration profile having a Mean Cₘₐₓ which is reduced by a percentage compared to the Mean Cₘₐₓ resulting from the b.i.d. administration of an immediate release dosage form which contains half the amount of the Pridopidine or a pharmaceutically acceptable salt thereof wherein the percentage is at least 5%. In another embodiment, the percentage is at least 10%. In another embodiment, the percentage is at least 20%. In another embodiment, the percentage is at least 30%. In another embodiment, the percentage is at least 40%. In another embodiment, the percentage is at least 50%. In another embodiment, the percentage is at least 60%. In another embodiment, the percentage is at least 70%. In another embodiment, the percentage is between 10% and 60%. In another embodiment, the percentage is between 20% and 50%. In another embodiment, the percentage is about 25%. In another embodiment, the percentage is about 35%. In another embodiment, the percentage is about 50%.

In an embodiment, the Mean time required to reach the maximal plasma, serum or blood concentration of the drug, following administration of the drug is more than 2 hours. In another embodiment, the Mean time required to reach the maximal plasma, serum or blood concentration of the drug, following administration of the drug is more than 4 hours.

In an embodiment, the pharmaceutically acceptable salt of Pridopidine is hydrochloride salt.

In another embodiment, the *in vivo* plasma profile is measured at steady state.

In an embodiment, the *in vivo* plasma profile is measured after single dose administration.

In an embodiment, the solid oral dosage form provides an *in vivo* plasma pridopidine concentration profile having a mean AUC_{0-inf} which is at least about 50% of the mean AUC_{0-inf} provided by the b.i.d. administration of an immediate release solid oral dosage form which contains half the amount of the Pridopidine or a pharmaceutically acceptable salt thereof.

In an embodiment, the solid oral dosage form provides an *in vivo* plasma pridopidine concentration profile having a mean AUC_{0-inf} which is at least about 55% of the mean AUC_{0-inf} provided by the b.i.d. administration of an immediate release solid oral dosage form which contains half the amount of the Pridopidine or a pharmaceutically acceptable salt thereof.

In an embodiment, the solid oral dosage form provides an *in vivo* plasma pridopidine concentration profile having a mean AUC_{0-inf} which is at least about 75% of the mean AUC_{0-inf} provided by the b.i.d. administration of an immediate release solid oral dosage form which contains half the amount of the Pridopidine or a pharmaceutically acceptable salt thereof.

In an embodiment, the solid oral dosage form releases not more than 50% of pridopidine after 1 hour when the oral dosage form is placed in a basket apparatus in 500mL of HCl 0.1N at a temperature of 37°C rotating at 100 revolutions per minute. In an embodiment, the solid oral dosage form releases not more than 75% of pridopidine after 3 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours. In another embodiment, the solid oral dosage form releases not less than 80% of pridopidine after 10 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours. In another embodiment, the solid oral dosage form releases not more than 30% of pridopidine after 2 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours. In another embodiment, the solid oral dosage form releases not more than 50% of pridopidine after 4 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours. In another embodiment, the solid oral dosage form releases not more than 65% of pridopidine after 6 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours. In another embodiment, the solid oral dosage form releases not less than 75% of pridopidine after 12 hours when the oral dosage form is placed in a basket apparatus in 500mL of HCl 0.1N at a temperature of 37°C rotating at 100 revolutions per minute for 120 minutes and then in buffer phosphate having a pH 6.8, for 12 hours.

In an embodiment, the dosage form is in the form of a capsule. In another embodiment, the dosage form is in the form of a tablet.

In an embodiment, the rate controlling excipient is a combination of two or more polymeric materials, and preferably the rate controlling excipient is a combination of at least a hydroxypropyl methylcellulose (HPMC) and hydrogenated castor oil.

In an embodiment, the polymeric material is hydroxypropyl methylcellulose. In another embodiment, the polymeric material is hydrogenated castor oil.

In an embodiment, the total amount of the rate controlling excipients is from about 8% to about 70% of the total weight of the dosage form, from about 10% to about 50% of the total weight of the dosage form, or from about 20% to about 50% of the total weight of the dosage form, from about 30% to about 50% or from about 30% to about 40% of the total weight of the dosage form.

In an embodiment, the polymeric material is between 10% and 50% by weight of the solid oral dose form.

In an embodiment, the polymeric material is between 20% and 50% by weight of the solid oral dose form. In another embodiment, the polymeric material is between 30% and 50% by weight of the solid oral dose form. In another embodiment, the polymeric material is between 30% and 40% by weight of the solid oral dose form. In another embodiment, the polymeric material is between 35% and 40% by weight of the solid oral dose form. In another embodiment, the polymeric material is at least 10% by weight of the solid oral dose form. In another embodiment, the polymeric material is at least 20% by weight of the solid oral dose form. In another embodiment, the polymeric material is at least 25% by weight of the solid oral dose form. In another embodiment, the polymeric material is at least 30% by weight of the solid oral dose form. In another embodiment, the polymeric material is at least 35% by weight of the solid oral dose form. In another embodiment, the polymeric material is at least 40% by weight of the solid oral dose form. In another embodiment, the polymeric material is about 37% by weight of the solid oral dose form. In another embodiment, the polymeric material is about 38% by weight of the solid oral dose form. In another embodiment, the polymeric material is about 40% by weight of the solid oral dose form.

In an embodiment, the modified release solid oral dosage form further comprises an ethylcellulose.

In an embodiment, the total amount of the ethylcellulose is from about 0.5% to about 10% of the total weight of the dosage form, from about 0.5% to about 7.2% of the total weight of the dosage form, from about 1.0% to about 5% of the total weight of the dosage form, from about 1.0% to about 3.0% of the total weight of the dosage form, from about 1.5% to about 3.0% of the total weight of the dosage form, or from about 1.5% to about 2.4% of the total weight of the dosage form.

In another embodiment, the ethylcellulose is about 1.5% by weight of the solid oral dose form. In an embodiment, the ethylcellulose is about 3.0% or about 2.4% by weight of the solid oral dose form.

In another embodiment, the polymeric material is hydroxypropyl methylcellulose, and wherein the hydroxypropyl methylcellulose is about 38% by weight of the solid oral dose form.

In an embodiment, the polymeric material is hydrogenated castor oil, and wherein the hydrogenated castor oil is about 38% by weight of the solid oral dose form.

In an embodiment, the polymeric material is hydroxypropyl methylcellulose, wherein the hydroxypropyl methylcellulose is about 37% by weight of the solid oral dose form, and wherein the ethylcellulose is between about 1.5% and about 3.0% by weight of the solid oral dose form.

In an embodiment, the weight ratio of the Pridopidine or the pharmaceutically acceptable salt thereof to the rate controlling excipient is from about 0.2:1 to about 1:1, preferably from about 0.3:1 to about 0.8:1, more preferably about 0.5:1 to about 0.7:1.

In an embodiment, the modified release solid oral dosage form further comprises a mucoadhesive.

In an embodiment, the mucoadhesive is selected from the group consisting of water soluble or water insoluble hydrophilic polymers, polymers that have swellable networks, hydrogels, and polymers with groups that can cross-link with other polymers or with a mucous membrane, preferably the mucoadhesive is polyethylene oxide.

In an embodiment, the Pridopidine or the pharmaceutically acceptable salt thereof comprises from about 15% to about 60% by weight of the dosage form. In another embodiment, the Pridopidine or the pharmaceutically acceptable salt thereof comprises from about 25% to about 50% by weight of the dosage form.

In an embodiment, the Pridopidine or the pharmaceutically acceptable salt thereof comprises about 25% by weight of the dosage form.

The present invention also provides a pharmaceutical formulation comprising the modified release solid oral dosage form, and one or more pharmaceutically acceptable carriers or excipients selected from a group consisting of: binder, filler, plasticizer, glidant and lubricant and mixtures thereof.

In an embodiment, the binder is selected from a group consisting of: starch, pregeletinized starch, polyethylene oxide, cellulose polymers, hydroxypropylmethyl cellulose, hydroxypropylcellulose, methylcellulose, hydroxyethyl cellulose, polyvinylpyrrolidone, polyvinyl alcohol and mixtures thereof.

In an embodiment, the filler is selected from a group consisting of: microcrystalline cellulose, sugar spheres, lactose, sorbitol, dextrose, sucrose, mannitol, dibasic or tribasic calcium phosphate, calcium sulfate, starch, retalac and mixtures thereof.

In an embodiment, the filler is microcrystalline cellulose and is a silicified microcrystalline cellulose.

In an embodiment, the filler is lactose. In another embodiment, the filler is a mixture of microcrystalline cellulose and lactose, and wherein the microcrystalline cellulose and is a silicified microcrystalline cellulose.

In an embodiment, the filler is between 5% and about 64% by weight of the solid oral dose form, between 10% and about 50% by weight of the solid oral dose form, between 15% and about 45% by weight of the solid oral dose form, between 20% and 40% by weight of the solid oral dose form, about 34% by weight of the solid oral dose form, about 16% by weight of the solid oral dose form, about 17% by weight of the solid oral dose form or about 18% by weight of the solid oral dose form.

In an embodiment, the filler is a mixture of silicified microcrystalline cellulose and lactose and wherein silicified microcrystalline cellulose is about 16% by weight of the solid oral dose form and lactose is about 17% or about 18% by weight of the solid oral dose form. In an embodiment, the plasticizer is selected from a group consisting of: polyethylene glycol, triethyl citrate, tributyl citrate, glycerin, dibutyl sebacate, triacetin, diethylphthalat and mixtures thereof.

In an embodiment, the glidant is selected from a group consisting of: starch, pregelatinized starch, silicone dioxide, colloidal silicone dioxide, talc and mixtures thereof.

In an embodiment, the glidant is colloidal silicone dioxide.

In an embodiment, the glidant is between 0.2% and about 4% by weight of the solid oral dose form, between 0.4% and about 3% by weight of the solid oral dose form, or between 0.43% and about 2.0% by weight of the solid oral dose form.

In an embodiment, the glidant is between 1.7% and about 4% by weight of the solid oral dose form, between 1.7% and about 3% by weight of the solid oral dose form, between 1.7% and about 2.0% by weight of the solid oral dose form, between 1.7% and 1.8% by weight of the solid oral dose form, about 1.7% by weight of the solid oral dose form or about 1.8% by weight of the solid oral dose form.

In an embodiment, the lubricant is selected from a group consisting of: sodium stearyl fumarate, stearic acid, magnesium stearate, calcium stearate, zinc stearate, talc, glyceryl behenate, glyceryl monostearate, and mixtures thereof.

In an embodiment, the lubricant is magnesium stearate.

In an embodiment, the lubricant is between 0.3% and about 4% by weight of the solid oral dose form, between 0.5% and about 3% by weight of the solid oral dose form, or between 1.1% and about 2.0% by weight of the solid oral dose form.

In an embodiment, the lubricant is between 1.7% and about 4% by weight of the solid oral dose form, between 1.7% and about 3% by weight of the solid oral dose form, between 1.7% and about 2.3% by weight of the solid oral dose form, between 1.8% and about 2.2% by weight of the solid oral dose form or about 2% by weight of the solid oral dose form.

The subject invention also provides the modified release solid oral dosage form or pharmaceutical formulation for use in the treatment of Huntington's Disease, Parkinson's disease, iatrogenic and non-iatrogenic Parkinsonism, dyskinesias, dystonias, Tourette's disease, iatrogenic and non-iatrogenic psychoses and hallucinoses, schizophrenia disorder or schizophreniform disorder, mood and anxiety disorders, manodepressive illness, depression, obsessive-compulsive disease, a sleep disorder, autism spectrum disorder, ADHD, age-related cognitive impairment, abuse of alcohol and substances used as narcotics, Alzheimer's disease or Retts syndrome, wherein the modified release solid oral dosage form or pharmaceutical formulation is adapted for once daily administration.

In an embodiment, two doses of the modified release solid oral dosage form or pharmaceutical formulation are administered to the individual and the interval between the two doses is about 24 hours.

In an embodiment, the subject is a human patient.

In an embodiment, the dosage form has the following *in vivo* plasma pridopidine concentration profile concentrations at steady state: a Cₘₐₓ from about 499 ng/ml to about 1400 ng/ml, a mean Cₘₐₓ from about from about 499 ng/ml to about 1157 ng/ml, or a mean Cₘₐₓ from about 906 ng/ml to about 1157 ng/ml. The invention also provides a method of treating an individual afflicted with a neurodegenerative disease or a disease related to dopamine, comprising once daily administration of the modified release solid oral dosage form or pharmaceutical formulation.

In an embodiment, the modified release solid oral dosage form or pharmaceutical formulation is adapted for once daily administration.

For the foregoing embodiments, each embodiment disclosed herein is contemplated as being applicable to each of the other disclosed embodiments. In addition, the elements recited in pharmaceutical composition embodiments can be used in the medical use embodiments described herein.

### Terms:

As used herein, the term "C" refers to the plasma/serum/blood concentration of an active pharmaceutical ingredient, or drug, following administration of the drug, e.g. Pridopidine, or a pharmaceutically acceptable salt thereof, in a biological sample, such as a patient sample (e.g., blood, plasma, serum, and cerebrospinal fluid). The concentration of the drug in the biological sample may be determined by any standard assay method known in the art. The term C includes such concentrations measurements as the Cₘᵢₙ, Cₘₐₓ, and Cₛₛ (average steady state concentration), and allows calculation of PK parameters such as AUC. Typically the term C refers to the plasma, serum or blood concentration.

As used herein, steady state refers to the situation in which the amount of drug eliminated at each dose interval equals the dose for that interval. In an embodiment, steady state administration as used herein is reached after 7 days. In an embodiment, steady state administration as used herein is reached after 9 days. In an embodiment, steady state administration as used herein is reached after 14 days.

As used herein, the term "Cₘₐₓ" refers to the maximum plasma, serum or blood concentration of a drug, following administration of the drug, e.g. Pridopidine, or a pharmaceutically acceptable salt thereof. Cₘₐₓ measured at steady state is sometimes referred as to C_{max,ss}. "Mean Cₘₐₓ" "C_{max,ss,}" and "mean Cₘₐₓ₀₋ₜ" are the mean of the respective Cₘₐₓ measured in a sample of patients. In an embodiment, the sample of patients includes four patients or more. Preferably, the sample should include ten patients or more.

As used herein, the term "Cₘᵢₙ" refers to the minimum plasma, serum or blood concentration of a drug, following administration of the drug, e.g. Pridopidine, or a pharmaceutically acceptable salt thereof. Cₘᵢₙ measured at steady state is sometimes referred as to C_{min,ss}.

As used herein, the term "Tₘₐₓ" refers to the time required to reach the maximal plasma, serum or blood concentration ("Cₘₐₓ") of the drug, following administration of the drug, e.g. Pridopidine, or a pharmaceutically acceptable salt thereof. Tₘₐₓ measured at steady state is sometimes referred as to T_{max,ss}.

As used herein, the term "AUC" refers to the area under the plasma, serum or blood concentration versus time curve.

As used herein, the terms "AUCₜ" and "AUC₀₋ₜ" refer to the area under the plasma, serum or blood concentration versus time curve wherein t is the last measured time point.

As used herein, the terms "AUC_{inf}", "AUC_{0-inf}" "AUC_{∞}", "AUC_{0-∞}" and AUC_{infinity} refer to the area under the plasma, serum or blood concentration versus time curve extrapolated to infinity.

As used herein, the terms "AUCₜₐᵤ" and "AUC₀₋ₜₐᵤ" refer to the area under the curve for a plasma, serum or blood concentration versus time curve of a drug over one dosing interval, following the administration of the drug such as Pridopidine or a pharmaceutically acceptable salt thereof. The area under the curve is measured for a time tau, where tau is the length of the dosing interval. The term AUC_{tau,ss} measures the exposure over the dosing interval at steady state. As use herein, tau is a 24 hours interval, this includes cases in which the drug is administered b.i.d. "Mean AUC," "Mean AUCₜ," "Mean AUC₀₋ₜ," "Mean AUC_{inf,}" "Mean AUCₜₐᵤ" and "Mean AUC₀₋ₜₐᵤ" are the mean of the respective AUC measured in a sample of patients. In an embodiment, the sample of patients includes four patients or more. Preferably, the sample should include ten patients or more.

As used herein, "single dose" administration means that the drug is administered over a 24 hours interval, either as once per day (qd) or twice a day (bid).

As used herein, the term "immediate release" or "IR" means that the escape or release in the body of a drug, such as Pridopidine or a pharmaceutically acceptable salt thereof, from a dosage form (tablet, capsule, pellet, etc.) occurs immediately or soon after administration, usually in minutes to a few hours. For example, 80% of drug may be dissolved over the first hour. The drug is released in a single action and the time of action of the drug is often limited.

As used herein, the term "modified release" or "MR" means that the escape or release of a drug, such as Pridopidine or a pharmaceutically acceptable salt thereof, from the dosage form (tablet, capsule, pellet, etc.) has been modified so that the release rate is slower than that in an unmodified or immediate release dosage form. Drug release takes place at a point in time after administration or for a prolonged period after administration or to a specific target in the body. Drug release may occur over several hours or over several days in order to maintain a therapeutically effective plasma concentration of the drug. Modified release encompasses delayed release (release at a time other than immediately after administration), extended release (release over a prolonged time period), sustained release (rate of drug release is sustained over a period of time), and controlled release (rate of drug release is controlled to get a particular drug concentration profile in the body).

As used herein, a slower dissolution profile is one in which the escape or release of a drug from the dosage form is slower, i.e. it takes more time for the drug to be released in a slower dissolution profile than a faster dissolution profile.

As used herein, the term "rate controlling excipient" refers to an excipient or a combination of excipients present in such amounts sufficient to reduce the rate of drug release from a dosage form, such as Pridopidine or a pharmaceutically acceptable salt thereof. A rate controlling excipient or a combination thereof controls the rate of drug release from a dosage form.

As used herein, the term "at least one pharmaceutically acceptable rate controlling excipient" or "one or more pharmaceutically acceptable rate controlling excipients" refers to the presence of one, two, three, four, or more rate controlling excipients in the dosage form.

As used herein, the term "Pridopidine" refers to Pridopidine free base. In certain embodiments, Pridopidine also includes any pharmaceutically acceptable salt, such as the HCl salt. Preferably, in any embodiments of the invention as described herein, the Pridopidine is in the form of its hydrochloride salt.

As used herein, an "amount" or "dose" of Pridopidine as measured in milligrams refers to the milligrams of Pridopidine base present in a preparation, regardless of the form of the preparation. A dosage of "90 mg Pridopidine" means the amount of Pridopidine base in a preparation is 90 mg, regardless of the form of the preparation. Thus, when in the form of a salt, e.g. a Pridopidine hydrochloride salt, the weight of the salt form necessary to provide a dose of 90 mg Pridopidine would be greater than Pridopidine mg due to the presence of the additional salt ion.

As used herein, a "unit dose", "unit doses" and "unit dosage form(s)" mean a single drug administration entity/entities.

As used herein, "about" in the context of a numerical value or range means ±10% of the numerical value or range recited or claimed.

As used herein, the term "once daily" means administering a dose once every 24 hours. As used herein, the term "QD" refers to a once daily administration.

As used herein, reference to a total weight of a dosage form refers to the total weight of a tablet (including any finishing coat), and in the case of a capsule, refers to the total weight of the capsule contents, excluding the weight of the capsule itself.

As used herein, the term "bioavailability" refers to the rate and extent to which an active pharmaceutical ingredient is absorbed from a dosage form and becomes available at the site of action.

A pharmacokinetic parameter or combinations of such parameters indicate the bioavailability of an active pharmaceutical ingredient, such as, Pridopidine following administration of Pridopidine or a pharmaceutically acceptable salt thereof. Such pharmacokinetic parameters are known to the person skilled in the art. Examples of such parameters include: Cₘₐₓ, AUC, AUCₜₐᵤ, and Tₘₐₓ.

The dosage forms of the present invention are formulated such that the pridopidine or a pharmaceutically acceptable salt thereof has an *in vitro* dissolution profile that is slower than that for an immediate release (IR) formulation. The dosage forms of the present invention may contain immediate release, sustained or extended release or delayed release components, or combinations thereof.

The pridopidine or a pharmaceutically acceptable salt thereof, in the solid oral dosage forms of the present invention can be provided in a modified release form such as modified, controlled or extended release (ER) form, with or without an immediate release (IR) component.

Modified release dosage forms can be made by, but not limited to, making pellets of different thicknesses so that the thinnest release the drug first and the thickest last, including a slow dissolving matrix or coating, including a non-dissolving coating around a tablet or capsule with small holes to let the drug out (by diffusion or solvation), controlling release of the drug by diffusion through a coating or matrix or by erosion of the matrix or coating by a process dependent on, for example, a particular condition such as the presence of enzymes or a particular pH. Modified release dosage forms have higher amounts of the drug than the amount present in an unmodified or immediate release dosage form.

The solid oral dosage forms of the present invention include all pharmaceutically acceptable salts of pridopidine. Preferably, the pridopidine is in its hydrochloride salt form.

The modified release solid oral dosage form of the present invention is suitable for administration in a one unit dosage form. Oral dosage forms for the purpose of the present invention include capsules, tablets, pellets, granules, powders coated or uncoated and combinations thereof. Optionally, if the dosage form is a capsule, the pridopidine or a pharmaceutically acceptable salt thereof is provided in the form of coated or uncoated pellets, granules, powders, mini tablets, tablets or capsules.

As used herein, a "polymeric material" includes any polymer. Any suitable polymeric material may be used in accordance with the teachings presented herein. The polymeric material may be any suitable shape and may take any suitable form.

The dosage forms of the present invention may include a mucoadhesives to slow the passage of the dosage form through the body so that the dosage form remains in the body sufficiently long for all the Pridopidine to be released in the body.

The solid oral dosage forms of the present invention can further comprise one or more mucoadhesives. Mucoadhesives slow the passage of the dosage form through the body so that the dosage form is inside the body during the interval between administrations so that pridopidine or a pharmaceutically acceptable salt thereof is released in the body. Mucoadhesives are substances that adhere to a biological tissue for an extended period of time by interfacial forces. The biological tissue is a mucous membrane. Mucoadhesion occur when a mucoadhesive contacts and adheres to a membrane by wetting of the mucoadhesive surface or from the swelling of the mucoadhesive. Further adhesion occurs when the mucoadhesive penetrates into the crevice of the membrane surface or when the chains of the mucoadhesive interacts with those of the mucus on the membrane. Suitable mucoadhesive are polymers that are water soluble or water insoluble hydrophilic polymers, polymers that have swellable networks, hydrogels, and polymers with groups that can cross-link with other polymers or with a mucous membrane.

The modified release solid oral dosage forms of the present invention can comprise at least one mucoadhesive with or without an immediate release component. For example, the dosage forms of the present invention can comprise at least one mucoadhesive with only an extended release component.

Silicified microcrystalline cellulose may be any commercially available form of this excipient, for example Prosolv^{®} SMCC 90.

Hydroxypropyl methylcellulose (HPMC) may be any commercially available form of this Hydrophilic carrier, for example Methocel^{™} K100 Premium CR, Methocel DC2, Benecel ME 233P.

Lactose spray dried (SD), Lactose anhydrous and Lactose monohydrate may be used interchangeable throughout this invention.

Colloidal silicon dioxide (CSD) is a fumed silica generally prepared by vapour-phase hydrolysis of a silicon compound, such as silicon tetrachloride. The product itself is usually a powder which is commercially available from a number of sources, including Degussa, Inc. (under the trade name Aerosil^{®}); Cabot Corporation (under the trade name Cab-O-Sil); Huber Engineered Materials (Huber GL100 and GL200); Wacker (Wacker HDK ^{®}); and E.I. DuPont & Co. Colloidal silicon dioxide is also known as colloidal silica, fumed silica, light anhydrous silicic acid, silicic anhydride, and silicon dioxide fumed, among others. A variety of commercial grades of CSD are produced by varying the manufacturing process.

Ethylcellulose may be added to the formulation in the form of dispersion for example, Surelease^{®}.

Pregelatinized Starch may be any commercially available form of this substance, for example Starch 1500^{®}.

LubriTose^{™} is Lactose plus between 2% and 10% Glyceryl MonoStearate (GMS), LubriTose^{™} Yellow contains 10% GMS and LubriTose^{™} blue contains 2% GMS.

Tablets may contain suitable binders, lubricants, disintegrating agents, coloring agents, flavoring agents, flow-inducing agents, melting agents, and plasticizers. For instance, for oral administration in the dosage unit form of a tablet or capsule, the active drug component can be combined with an oral, non-toxic, pharmaceutically acceptable, inert carrier such as xylose, gelatin, agar, starch, methyl cellulose, dicalcium phosphate, calcium sulfate, mannitol, sorbitol, microcrystalline cellulose and the like. Suitable binders include starch, gelatin, natural sugars such as corn starch, natural and synthetic gums such as acacia, tragacanth, or sodium alginate, povidone, polyvidone, carboxymethylcellulose, hydroxypropyl cellulose, polyethylene glycol, waxes, and the like. Glidants used in these dosage forms include silicon dioxide and the like. Lubricants used in these dosage forms include sodium oleate, sodium stearate, sodium benzoate, sodium acetate, stearic acid, sodium stearyl fumarate, talc and the like. Disintegrators include, without limitation, starch, methyl cellulose, agar, bentonite, xanthan gum, croscarmellose sodium, sodium starch glycolate and the like, suitable plasticizers include triacetin, triethyl citrate, dibutyl sebacate, polyethylene glycol and the like.

The modified release solid oral dosage forms of the present invention may further comprise one or more pharmaceutically acceptable carriers or excipients.

Examples of pharmaceutical acceptable excipients are fillers, binders, glidants, plasticizer and lubricants.

Tablets in accordance with this invention can be prepared by conventional mixing, comminution, and tabletting techniques that are well known in the pharmaceutical formulations industry. The modified release tablet, for example, may be obtained by direct compression by punches and dies fitted to a rotary tabletting press, ejection or compression molding, dry of wet granulation followed by compression, or forming a paste and extruding the paste into a mold or cutting the extrudate into short lengths. Preferably, the process used for preparing tablets is direct compression of the blend.

Compression can be accomplished using conventional equipment. Typically, the blend of active ingredients with or without excipients is passed through a roller apparatus for compaction. However, other means for compacting the API mixture, e.g., compaction into slugs (or "slugging"), may be used.

To achieve the desired modified release rates, the modified release dosage form may be formulated as a polymeric coating or matrix.

USP #1 apparatus (basket), is the apparatus 1 described in the United States Pharmacopeia, 29th Edition, chapter 711. The apparatus may be constructed as follows:
The assembly consists of the following: a covered vessel made of glass or other inert, transparent material; a motor; a metallic drive shaft; and a cylindrical basket. The vessel is partially immersed in a suitable water bath of any convenient size or placed in a heating jacket. The water bath or heating jacket permits holding the temperature inside the vessel at 37 ± 0.5 during the test and keeping the bath fluid in constant, smooth motion. No part of the assembly, including the environment in which the assembly is placed, contributes significant motion, agitation, or vibration beyond that due to the smoothly rotating stirring element. Apparatus that permits observation of the specimen and stirring element during the test is preferable. The vessel is cylindrical, with a hemispherical bottom and with one of the following dimensions and capacities: for a nominal capacity of 1 L, the height is 160 mm to 210 mm and its inside diameter is 98 mm to 106 mm; for a nominal capacity of 2 L, the height is 280 mm to 300 mm and its inside diameter is 98 mm to 106 mm; and for a nominal capacity of 4 L, the height is 280 mm to 300 mm and its inside diameter is 145 mm to 155 mm. Its sides are flanged at the top. A fitted cover may be used to retard evaporation. The shaft is positioned so that its axis is not more than 2 mm at any point from the vertical axis of the vessel and rotates smoothly and without significant wobble. A speed-regulating device is used that allows the shaft rotation speed to be selected and maintained at the rate specified in the individual monograph, within ±4%. Shaft and basket components of the stirring element are fabricated of stainless steel type 316 or equivalent.

Unless otherwise specified in the individual monograph, use 40-mesh cloth. A basket having a gold coating 0.0001 inch (2.5 µm) thick may be used. The dosage unit is placed in a dry basket at the beginning of each test. The distance between the inside bottom of the vessel and the basket is maintained at 25 ± 2 mm during the test.

### Pridopidine

Pridopidine is absorbed relatively rapidly after oral administration with tₘₐₓ between 0.5 to 4 hours (Lindskov 2012). After absorption, pridopidine is eliminated partly by urinary excretion, partly by hepatic metabolism, and primarily by N-depropylation via the CYP2D6 pathway into one main inactive metabolite, 4-(3-(methylsulfonyl)phenyl)piperidine, with an elimination half-life after repeated doses of 10-14 hours. CYP2D6 polymorphisms can be classified according to one of four levels of activity: poor metabolizers (PMs), intermediate metabolizers (IMs), extensive metabolizers (EMs), and ultrarapid metabolizers (UMs). The EM phenotype is expressed by the majority of the population (around 90%). Approximately 5-10% of the Caucasian European and North American population, and 1% of Chinese, Japanese and Korean populations are PMs. PMs inherit two deficient CYP2D6 alleles and, as a result, metabolize drugs at a notably slower rate. The Ultrarapid metabolizers (UM) phenotype is caused by the duplication, multiduplication, or amplification of active CYP2D6 genes, including primarily the CYP2D6^{∗}2 allele, but also involving CYP2D6^{∗}1 and others. Individuals with the UM phenotype metabolize drugs at an ultrarapid rate. Lastly, individuals who are heterozygous for a defective CYP2D6 allele often demonstrate an IM phenotype with a wide spectrum of metabolic activity that can range from marginally better than the PM phenotype to activity that is close to that of the EM phenotype (Bernard 2006).

A Phase 2, Dose-Finding, Randomized, Parallel-Group, Double- Blind, Placebo-Controlled Study, Evaluating the Safety and Efficacy of Pridopidine 45 mg, 67.5 mg, 90 mg, and 112.5 mg Twice-Daily Versus Placebo for Symptomatic Treatment in Patients With Huntington's Disease is planned (Clinicaltrials.gov Clinical Trial Identifier NCT02006472). Therefore, a dosage form comprising Pridopidine at these doses with a good safety profile is desirable. In addition, a dosage form administered less frequently than twice a day would increase compliance and would be preferable for the patients and caregivers.

The present invention is illustrated by the following examples, which are not intended to limit the scope of the invention.

### Examples

### Example 1: Safety of Pridopidine Administration Following Administration of Immediate Release dosage forms.

### Multiple Ascending Dose (MAD) study

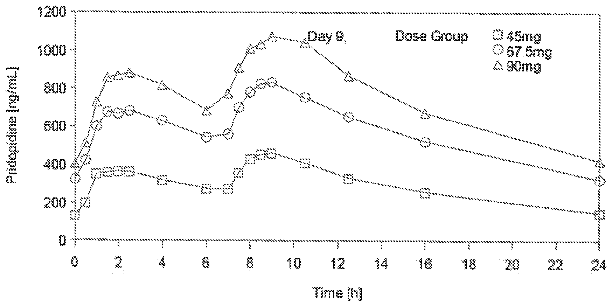

In a Multiple Ascending Dose (MAD) study, thirty-six (36) healthy volunteers of both sexes (age 18-55 years) from the CYP2D6 EM genotype were randomized to 3 cohorts. Within each cohort, 9 subjects were randomized to 2 ascending doses of pridopidine b.i.d. in fixed sequence (45-67.5mg, 67.5-90mg, and 90-112.5mg), and 3 subjects to matching placebo b.i.d. treatment in both treatment periods. Each period consisted of 9 consecutive days of b.i.d. dosing (with a 6.5 hr interval between the morning and the afternoon dose) to steady state (Østerberg 2012). Pridopidine drug concentrations were monitored up to 24 hours after the first dose and single dose parameters (associated with the first 24 hours interval) were determined. The geometric mean plasma concentrations versus time during the study are presented in Figure 1.

Safety and tolerability were assessed by monitoring adverse events (AEs), measuring vital signs, electrocardiograms (ECGs), and clinical laboratory values. PK parameters of pridopidine were calculated using non-compartmental methods and summarized by descriptive statistics by treatment/dose level (Table 1A and 1B for Day 9 and Day 1 respectively). The dosing interval in this trial (tau) was defined as 24 hours.

**Table 1A: Summary of pharmacokinetic parameters at sicady state (mean ± SD)**

| **N** | **Dose and Regimen** | **Mean ± SD** | | | |
|---|---|---|---|---|---|
| | | **AUCtau,ss (hr*ng/mL)** | **Cmax,ss (ng/mL)** | **T1/2 (h)** | **Tmax,ss (h) (range)** |
| 8 | | 7178 | 499 | 10.5 | 1.5 |
| | IR 45mg BID | ±1672 | ±97 | ±3.05 | (1.0-2.5) |
| | | 5253-10458 | 382-664 | | |
| 16 | | 14185 | 906 | 10.4 | 2.0 |
| | IR 67.5mg BID | ±3747 | ±207 | ±2.5 | (1.0-4.0) |
| | | 10228-21065 | 639-1287 | | |
| 14 | | 18065 | 1157 | 10.2 | 2.0 |
| | IR 90mg BID | ±3413 | ±190 | ±2.1 | (1.0-4.0) |
| | | 12670-24151 | 871-1568 | | |

**Table 1B: Summary of pharmacokinetic parameters after single dose administration (mean± SD)**

| **N** | **Dose and Regimen** | **Mean ± SD** | | **Median** | |
|---|---|---|---|---|---|
| | | **AUC_{0-inf} (hr*ng/mL)** | **Cmax,_{6.5-24} (ng/mL)** | **T1/2 (h) (range)** | **Tmax,ss (h) (range)** |
| 8 | IR 45mg BID | 5043 | 327 | 6.41 | 1.0 |
| | | ±3276 | ±99.3 | (4.31-15.4) | (1.00-2.50) |
| | | 2249-12570 | 244-545 | | |
| 16 | IR 67.5mg BID | 7897 | 486 | 7.40 | 1.5 |
| | | ±2811 | ±116 | (4.39-11.2) | (1.00-2.50) |
| | | 3907-14620 | 324-813 | | |
| 14 | | 13594 | 718 | 9.00 | 1.75 |
| | IR 90mg BID | ±3880 | ±144 | (6.61-14.0) | (1.00-2.50) |
| | | 7934-22138 | 493-1002 | | |

As shown in Table 2, the adverse events, such as gastrointestinal disorders, increased in frequency with increasing doses. Psychiatric disorders were primarily observed at the 90 mg dose b.i.d., with one observation of psychiatric disorder in the 45 mg dose b.i.d.

A prolonged QT interval has been associated with increased risks for Torsade de Points. Electrocardiogram (ECG) measurements were collected at baseline (predose on the 1^{st} day) and serially on Day 9 (coupled to the PK samples). A high precision QT measurement technique was implemented. The primary endpoint for the QTc analysis was placebo-corrected change-from-baseline QTcF (QT corrected through the Fredericia correction;, ΔΔQTcF). The relationship between pridopidine plasma concentrations and ΔΔQTcF was quantified using a linear mixed-effects modeling approach.

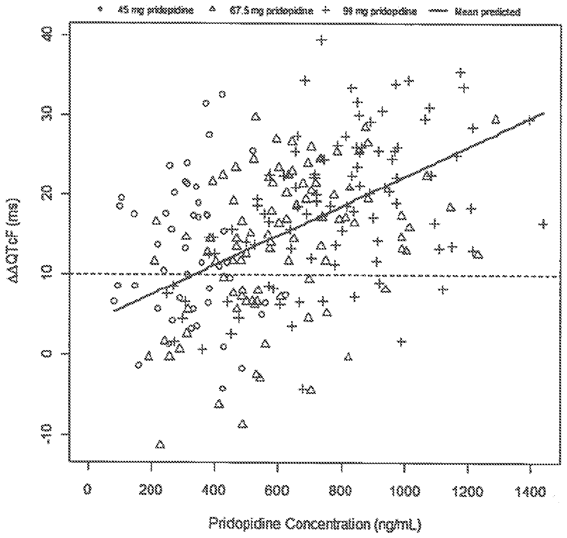

The results showed a concentration-dependent effect of pridopidine on ΔΔQTcF, suggesting that higher concentrations result in longer QT prolongation. The estimated population intercept and slope was 3.82 ms and 0.0185 ms per ng/mL (CI: 0.0139 to 0.0231), respectively (Figure 2).

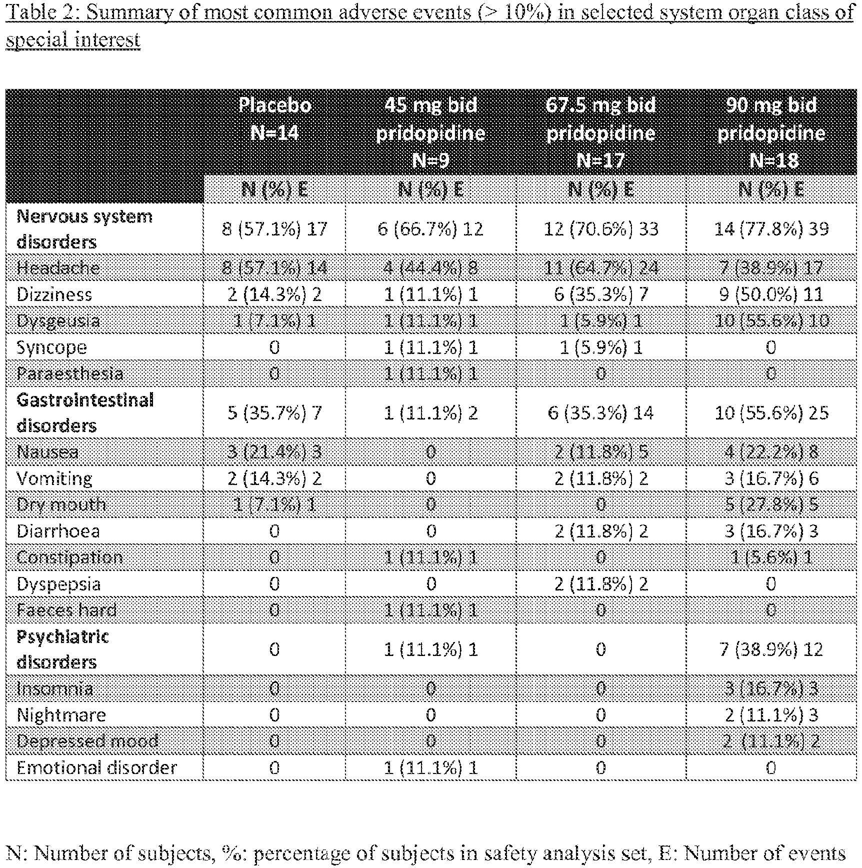

### Summary of the Results of Example 1

The results as presented in Table 1A showed that a mean C_{max,ss} as high as about 1157 ng/ml (with a maximal measured value of 1568 ng/ml), can be safely administered to humans. The results presented in Table 1A also shows that the 45 mg IR bid administration resulted in a mean C_{max,ss} value of 499 ng/ml and mean AUC_{tau,ss} value (tau defined as a 24 hours interval covering two doses) of 7178 hr^{∗}ng/mL; these values are known to show therapeutic benefit. The range of AUC_{tau,ss} resulting from the administration of 45-90mg b.i.d was 5253-24151 hr^{∗}ng/mL. Similarly, the results as presented in Table 1B showed that a mean Cₘₐₓ as high as about 718 ng/ml at day 1 (with a maximal measured value of 1002 ng/ml), can be safely administered to humans. The results presented in Table 1B also shows that the 45 mg IR bid administration resulted in a mean Cₘₐₓ value of 327 ng/ml and mean AUC_{0-inf} value of 5043 hr^{∗}ng/mL. The range of AUC_{0-inf} resulting from the administration of 45-90mg b.i.d was 2249-22138 hr^{∗}ng/mL.

Additionally, the results presented in Figure 2 shows that a concentration as high as 1400 ng/ml can be considered safe related to the potential prolongation of the QT interval.

The results in Tables 1A, 1B, and 2 show that when certain dosages of pridopidine are administered, there is a risk of increasing the frequency of adverse events in comparison to the frequency of adverse events in previously tested safe dosages of pridopidine. The adverse events include, but are not limited to, QT interval prolongation, gastrointestinal disorders, and psychiatric disorders. The problem to be solved by this application is to provide new formulations of high dose pridopidine which reduce the frequency of the adverse events. By preventing the Cₘₐₓ from reaching very high values, applicants can limit the adverse events, such as those shown in Example 1. It was not known that one should prevent the Cₘₐₓ of pridopidine from peaking in order to minimize some or all adverse events related to a pridopidine dose. With the understanding of the problem, applicants invented the present invention, a modified release dosage form of pridopidine which prevents the Cₘₐₓ from rising above previously tested safe doses.

### Example 2: Pridopidine dosage forms.

Dosage forms comprising 90mg Pridopidine were formulated and the in vitro dissolution rate was tested.

Dosage forms comprising 101.6mg Pridopidine HCl (equivalent to 90mg Pridopidine base) were formulated by matrix mechanism using excipients in combination with several hydrophilic (water-soluble) and/or hydrophobic (water-insoluble) carriers.

A hydrophilic matrix, modified release system is a dynamic one involving polymer wetting, polymer hydration, gel formation, swelling and polymer dissolution. The rate of the drug release is determined by diffusion (if soluble) through the gel and by the rate of tablet erosion. At the same time, other soluble excipients or drugs will also wet, dissolve and diffuse out of the matrix while insoluble materials will be held in place until the surrounding polymer/excipient/drug complex erodes or dissolves away.

### Manufacture of Modified Release (MR) Pridopidine dosage forms

A matrix tablet was prepared by wet granulation method. A granule was prepared to be used in combination with carrier or carriers and selected excipients for obtaining modified release formulations.

### Manufacture of pridopidine granulates:

High Shear Granulation: All granulation ingredients were added to the granulator bowl and pre-blend (chopper at medium/high speed; impeller at medium/low speed) for a sufficient time to ensure mixture uniformity and to break-up any agglomerates. Granulations liquid was added and blend (chopper at high speed; impeller at medium speed). The quantity of granulation fluid required is highly formulation dependent. The granules were dried using a fluid bed dryer and milled by Quadro Comill.

Granules of 90mg and high dose pridopidine are presented in Table 3.1, Table 3.2, and Table 3.3, respectively.

**Table 3.1: Composition of Granules R1-R3**

| **Batch No.** | **Use** | **R1** | **R2** | **R3** |
|---|---|---|---|---|
| **Composition** | - | **mg/tab** | **mg/tab** | **mg/tab** |
| Pridopidine HCl | Drug Substance | 101.6 | 101.6 | 101.6 |
| Ethylcellulose (Ethocel^{™} 7 Premium) | Binder | 20.4 | 20.4 | 50.8 |
| CaHPO₄ | Insoluble filler | - | 178.0 | 101.6 |
| Pregelatinized Starch (Starch 1500^{®}) | Filler, disintegrant, binder | - | - | 50.8 |
| Total Weight | - | 122.0 | 300.0 | 304.8 |

**Table 3.2: Composition of Granules R4 based on HD IR Capsules formulation**

| **Batch No.** | **Use** | **R4** |
|---|---|---|
| **Composition** | **-** | **mg/Tab** |
| Pridopidine HCl | Drug Substance | 127.0 |
| Microcrystalline Cellulose (Avicel PH 102) | Diluent/disintegrant | 65.0 |
| Hydroxypropyl Cellulose (Klucel) | Binder | 10.0 |
| Total Weight | - | 202.0 |

**Table 3.3: Composition of Granules R5**

| **Batch No.** | **Use** | **R5** |
|---|---|---|
| **Composition** | **-** | **mg/Tab** |
| Pridopidine HCl | Drug Substance | 101.6 |
| Silicified Microcrystalline Cellulose (Prosolv^{®} SMCC 90) | Filler | 63.2 |

### Dissolution test of the dosage forms:

A typical dissolution for Pridopidine tablets uses an USP #1 apparatus (basket), rotating at 100 RPM and 37°C in 500mL of HCl 0.1N for 2 hours and then in buffer phosphate pH 6.8, for 12 hours. The buffer phosphate is prepared by dissolving 6.805 g of KH2PO4 phosphate dibasic and 4.48mL 5M NaOH, diluted to 1000mL with deionized water and mixed thoroughly. The sample is tested by UV detector set at 268 nm and then returned to the dissolution vessel. The same dissolution results were obtained using an USP #2 apparatus (paddle) at 75 RPM.

### Example 3: Modified Release (MR) Pridopidine dosage forms of the invention.

Dosage forms were formed with the R5 granulate and are included within the invention. Dosage forms included within the invention are presented in Table 4.

**Table 4: Dosage Forms included in the present invention**

| **Formulation Ingredients** | **Composition (mg)/Prototype No.** | | |
|---|---|---|---|
| | **MR-1** | **MR-2** | **MR-3** |
| Pridopidine HCl | 101.6 | 101.6 | 101.6 |
| Silicified Microcrystalline Cellulose (Prosolv^{®} SMCC 90) | 63.2 | 63.2 | 63.2 |
| Hydroxypropyl methylcellulose (Methocel ^{™} K100M Premium CR) | ** | 150.0 | 150.0 |
| Hydrogenated Castor Oil (HCO) | 150.0 | ** | ** |
| Lactose SD | 70.0 | 70.0 | 70.0 |
| Colloidal Silicon Dioxide (Aerosil^{®}) | 7.2 | 7.2 | 7.2 |
| Magnesium Stearate | 8.0 | 8.0 | 8.0 |
| Ethylcellulose (Surelease^{®}) | ** | ** | 6.0-12.0 |
| Total | 400.0±5%^{∗} | 400.0±5% | 406.0-412.0±5% |

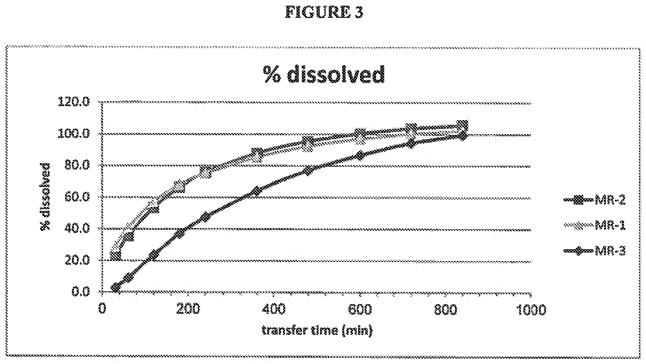

The in vitro dissolution results are presented in Table 5 and Figure 3.

**Table 5: In vitro dissolution profile of dosage forms MR-1, MR-2. and MR-3**

| Sampling time (min) | pH | % dissolved | | |
|---|---|---|---|---|
| | | MR-1 | MR-2 | MR-3 (8mg Ethylcellulose/tablet) |
| 60 | 1.2 | 41 | 35 | 9 |
| 120 | 1.2 | 57 | 54 | 24 |
| 180 | 6.8 | 68 | 67 | 37 |
| 240 | 6.8 | 75 | 76 | 48 |
| 360 | 6.8 | 86 | 88 | 64 |
| 480 | 6.8 | 92 | 96 | 77 |
| 600 | 6.8 | 97 | 101 | 86 |
| 720 | 6.8 | | | 94 |

In addition to the dissolution profiles of formulations determined shortly after formation (T0), dissolution profiles of formulations MR-1, MR-2 and MR-3 were also determined after 3 months (3M) and 4 month (4M), as presented in Table 5A. In addition, the dissolution profiles of different batches were determined, and are also presented in Table 5A. For each formulation, Batch 1 is the same batch presented in Table 5.

**Table 5A: Dissolution profile of different batches of formulations MR-1, MR-2 and MR-3**

| MR-1 | | | | | | | |
|---|---|---|---|---|---|---|---|
| Time | Time | Batch 1 | | | Batch 2 | Batch 3 | Batch 4 |
| hours | min | T0 | 3M | 4M | T0 | T0 | T0 |
| 1 | 60 | 41 | 33 | 34 | 36 | 35 | 34 |
| 2 | 120 | 57 | 46 | 49 | 50 | 49 | 47 |
| 3 | 180 | 68 | 60 | 65 | 60 | 58 | 56 |
| 4 | 240 | 75 | 67 | 72 | 68 | 65 | 63 |
| 6 | 360 | 86 | 77 | 82 | 79 | 75 | 73 |
| 8 | 480 | 92 | 84 | 89 | 87 | 83 | 81 |
| 10 | 600 | 97 | 89 | 95 | 92 | 89 | 87 |
| 12 | 720 | 100 | 92 | 99 | 96 | 93 | 92 |
| 14 | 840 | 102 | 95 | 101 | 99 | 96 | 96 |

| MR-2 | | | | | | | |
|---|---|---|---|---|---|---|---|
| Time | Time | Batch 1 | | | Batch 2 | Batch 3 | Batch 4 |
| hours | min | T0 | 3M | 4M | T0 | T0 | T0 |
| 1 | 60 | 36 | 31 | 31 | 36 | 28 | |
| 2 | 120 | 54 | 46 | 46 | 53 | 42 | 28 |
| 3 | 180 | 67 | 61 | 62 | 66 | 55 | 42 |
| 4 | 240 | 76 | 69 | 72 | 76 | 65 | 54 |
| 6 | 360 | 88 | 81 | 87 | 89 | 78 | 63 |
| 8 | 480 | 96 | 88 | 92 | 97 | 88 | 75 |
| 10 | 600 | 101 | 93 | 97 | 103 | 94 | 83 |
| 12 | 720 | 104 | 96 | 100 | 107 | 98 | 88 |
| 14 | 840 | 106 | 98 | 102 | 109 | 100 | 92 |

| MR-3 | | | | | | | |
|---|---|---|---|---|---|---|---|
| Time | Time | Batch 1ⁱ | Batch 2" | Batch 3ⁱⁱⁱ | | | |
| hours | min | T0 | T0 | T0 | 3M | | |
| 1 | 60 | 9 | 5 | 6 | 16 | | |
| 2 | 120 | 24 | 15 | 18 | 30 | | |
| 3 | 180 | 37 | 26 | 31 | 45 | | |
| 4 | 240 | 48 | 35 | 42 | 55 | | |
| 6 | 360 | 64 | 50 | 58 | 71 | | |
| 8 | 480 | 77 | 63 | 72 | - | | |
| 10 | 600 | 87 | 73 | - | 90 | | |
| 12 | 720 | 94 | 82 | 90 | 97 | | |
| 14 | 840 | 100 | 89 | - | - | | |

| | | | | | | | |
|---|---|---|---|---|---|---|---|
| ^{i.} 8mg Ethylcellulose/tablet " 10mg Ethylcellulose/tablet ^{iii.} 7mg Ethylcellulose/tablet | | | | | | | |

### Example 4: Immediate Release (IR) Pridopidine dosage forms

In comparison to the MR Pridopidine dosage forms, the IR dosage forms of Pridopidine were almost totally dissolved after about 20-30 minutes. Exemplary dissolution profiles of IR dosage forms of Pridopidine are presented in Table 5.1. The composition of the IR dosages forms in Table 5.1 are presented in Table 5.2:

**Table 5.1: Dissolution development for pridopidine capsules - IR Dosage Forms**

| **Strength** | **Batch** | **pH** | **% dissolved** | | | | | |
|---|---|---|---|---|---|---|---|---|
| **(mg)** | | | **5 min** | **10 min** | **15 min** | **20 min** | **30 min** | **60 min** |
| 22.5 | AA | 1.2 | 62.1 | 97.5 | 99.4 | 99.4 | 99.9 | 99.6 |
| 22.5 | AA | 4.0 | 38.8 | 96.3 | 98.1 | 98.3 | 98.3 | 98.5 |
| 22.5 | AA | 6.8 | 65.3 | 94.1 | 96.1 | 96.7 | 96.9 | 97.0 |
| 45 | DD | 1.2 | 30.0 | 83.3 | 93.5 | 96.9 | 98.3 | 98.4 |
| 45 | DD | 4.0 | 30.3 | 82.3 | 94.0 | 97.4 | 97.7 | 97.8 |
| 45 | DD | 6.8 | 23.4 | 67.4 | 94.0 | 96.6 | 96.8 | 96.6 |

**Table 5.2: Composition of IR Dosage Forms**

| | **Batch No.** | **AA** | **DD** |
|---|---|---|---|
| **Formulation** | Use | **Composition** mg/capsule | |
| Pridopidine HCl | Drug Substance | 25.4 | 50.8 |
| Silicified Microcrystalline Cellulose (Prosolv^{®} SMCC 90) | Filler | 43.2 | 86.4 |
| Magnesium Stearate | Lubricant | 1.4 | 2.8 |

Additional formulations developed during the development of the formulations of the present invention are presented in Table 5.3. As can be seen, formulation A was formulated without a carrier. Some formulations, such as formulation B, C and D, included rate controlling excipients (Table 5.3).

The dissolution profiles of formulations A, B, C and D are presented in Table 5.4. As shown in Table 5.4, formulation A provides immediate release of drug substance (1 hour).

The presence of up to 16% of hydrophobic carrier Hydrogenated Castor Oil (HCO) in matrix tablets with (formulation B) or without (formulation C) soluble filler (Lactose), did not result in delayed release of Pridopidine, which was released after approximately 1 hour in both cases.

Dissolution results of formulation D showed that 10% hydrophobic carrier (HCO) in formulation D also provided 1 hour release of Pridopidine.

**Table 5.3 Formulations with different carriers and carrier amounts**

| **Batch No.** | **Use** | **A** | **B** | **C** | **D** |
|---|---|---|---|---|---|
| **Composition** | - | mg/Tablet | mg/Tablet | mg/Tablet | mg/Tablet |
| Granules¹ | - | **R2** 300.0 | **R3** 304.8 | **R3** 304.8 | **R4** 202.0 |
| Hydroxypropyl methylcellulose (Methocel ^{™} K100M Premium CR) | Hydrophilic carrier | ^{∗} | ^{∗} | * | * |
| Hydrogenated Castor Oil (HCO) | Hydrophobic carrier | ^{∗} | 60.0 | 60.0 | 23.0 |
| Lactose (Anhydrous) | Soluble filler | ^{∗} | 75.2 | ^{∗} | ^{∗} |
| Microcrystalline Cellulose and Glyceryl Monostearate (Lubritose^{™} Blue) | Soluble filler | ^{∗} | ^{∗} | ^{∗} | ^{∗} |
| Colloidal Silicon Dioxide (Aerosil) | Flow agent or glidant | 5.0 | 5.0 | 5.0 | 1.0 |
| Magnesium Stearate | Lubricant | 5.0 | 5.0 | 5.2 | 2.5 |
| Tablet Weight | | 310.0 | 450.0 | 375.0 | 228.5 |
| **Dissolution profile²** | | **1h release** | **1h release** | **1h release** | **1h release** |

| | | | | | |
|---|---|---|---|---|---|
| ¹ Granules R2, R3, and R4 are listed in Table 3.1, or 3.2. ² Dissolution testing was performed using USP, apparatus I at 100rpm, in 900mL purified water at 37°C. | | | | | |

**Table 5.4 Dissolution Profiles of the Formulations in Table 5.3**

| | **Time/ Batch No.** | **5min** | **1h** | **3h** | **6h** | **9h** | **12h** |
|---|---|---|---|---|---|---|---|
| Release rate (%) | A | 23 | 101 | 109 | | | |
| | B | 18 | 97 | | | | |
| | C | 18 | 106 | | | | |
| | D | 22 | 88 | 100 | | | |

### Summary of Examples 2-4

The exemplified dosage forms presented in Example 3 (Table 5) showed an *in vitro* dissolution profile wherein about 41% (MR-1), about 36% (MR-2) and as low as about 9% (MR-3) were dissolved in the first hour. After 4 hours, about 75% (MR-1), about 76% (MR-2), and about 48% (MR-3) of the Pridopidine were dissolved. Even after ten hours, not all the Pridopidine in the dosage form MR-1 was dissolved, and only 86% of the Pridopidine included in dosage form MR-3 was dissolved, in comparison to IR dosage forms of Pridopidine shown in Example 4, where more than 20% of pridopidine was already dissolved after 5 minutes, and were almost totally dissolved after about 20-60 minutes. As shown in Example 4, some formulations containing rate controlling excipients were found not to act as modified release formulation.

### Example 5: Development of a pharmacokinetic model useful for the simulation of PK profile following Pridopidine administration.

PK plasma profiles resulting from administration of the dosage forms were calculated using a simulation program. The PKPlus^{™}module portion of Gastroplus^{™} simulator software available from Simulations Plus, Incorporated, was first used to determine the best type of ACAT (Advanced Compartmental Absorption and Transit) model for immediate release pridopidine dosing.

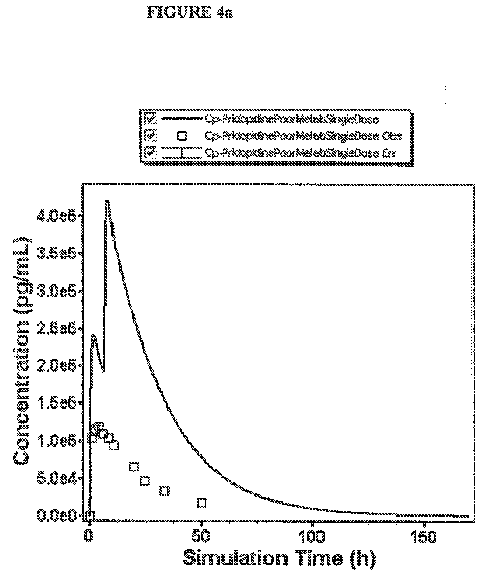

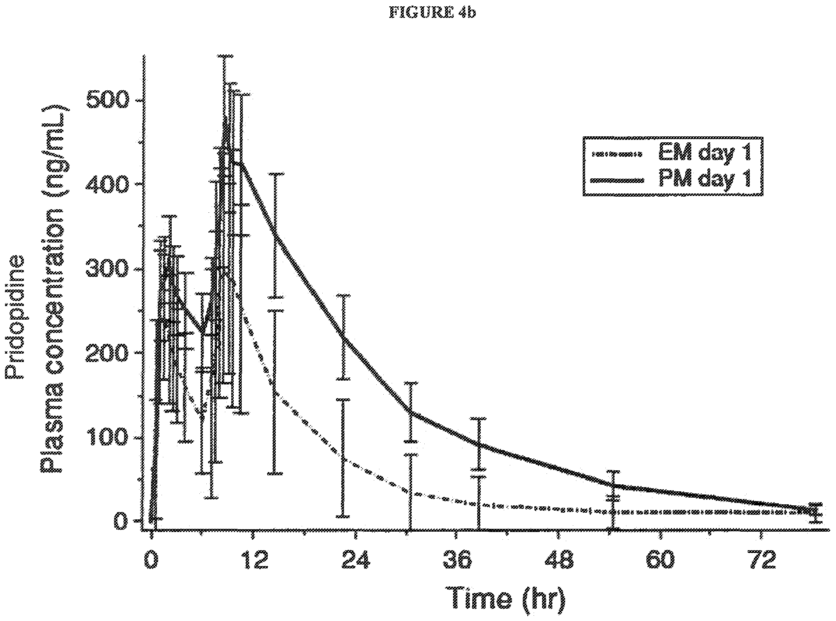

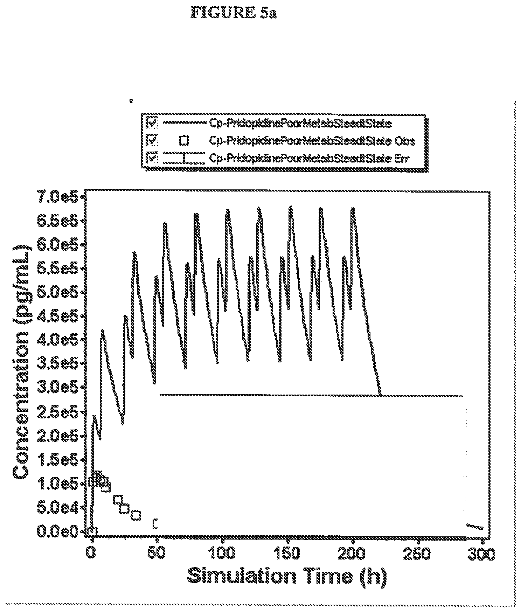

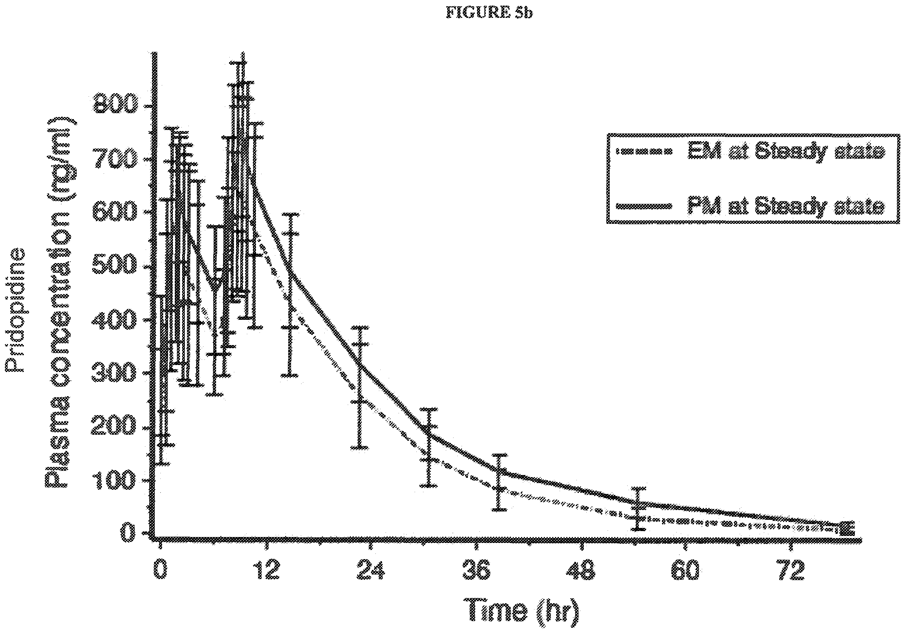

Concentration data obtained following administration of an immediate release of pridopidine (IR) were used as an approximation for IV. The IR data was obtained from the study published by Helldén et al. (2012). Pridopidine was dosed as either 25.4mg pridopidine HCl (22.5mg pridopidine base) or 50.8 mg pridopidine (45mg pridopidine base) of an IR capsule to poor metabolizers (PM) and extensive metabolizers (EM), respectively. PK samples were taken over 50 hours post-dose. Mean plasma concentration vs. time data for the PM group after single dose were extracted using summary graphs from UN-SCAN-ITTM graph digitizing software available from Silk Scientific Inc. Plasma concentration for the following time points was inputted into the PK Plus module in hours: 0.0, 0.9, 2, 3, 4, 6, 9, 10.6, 19.7, 25, 33, and 50 hours. The PK Plus module portion estimated mean pharmacokinetic parameters and performed calculations for the goodness of fit and Akaike Information Criterion for Noncompartment, One-Compartment, Two-Compartment and Three-Compartment Models. Based on the lowest Akaike information criterion value, the two compartment model was selected as having the best fit. The model was validated by comparison to data from another Pridopidine study (Linskov 2013, Hellden 2012) as presented in Figures 4 and 5.

The model can simulate plasma concentration of poor metabolizers (PM) of Pridopidine administered with a single dose as well as multiple doses (steady state). Importantly, it has been shown that during multiple dose administration pridopidine can inhibit its own CYP2D6-driven metabolism in EM subjects, meaning that upon repeated dosing, PMs and EMs exhibit comparable exposure due to a reduction in CYP2D6-related pridopidine metabolism in EMs over time (Lindskov 2012). In relation to presented model, this means that while simulation of plasma concentrations following single dose administrations would be relevant to PMs only, results of the steady state PM can be applied to the steady state in EMs, and therefore a general population including both EMs and PMs. For the same reasons, the model is expected to fit the UM and IM phenotypes as well.

### Example 6: Predicted PK parameters following administration of the oral dosage forms.

Using the dissolution profiles of the dosage forms described in Examples 2-4, and the pharmacokinetic model described in Example 5, the predicted plasma concentrations resulting from multiple administrations of MR dosage forms of pridopidine were calculated. Pharmacokinetic parameters were calculated for twice daily (b.i.d.) administration of IR formulations containing different doses of Pridopidine (with a 6.5h interval and a 7h interval between doses), and once daily administration of modified release dosage forms MR-1, MR-2, and MR-3 containing different doses of Pridopidine, both after single dose administration and at steady state. Data from 45 to 157.5 mg IR administered b.i.d. or MR dosage forms (90 to 315mg) administered once daily are presented in Table 6 (day 1) and Table 7 (steady state). The simulation calculated a PK value equivalent to mean Cₘₐₓ.

**Table 6: Observed and Simulated Pridopidine PK parameters on day 1 following a single dose of IR dosage forms b.i.d or MR dosage forms QD**

| Sample Name | AUC day 10-38h^{∗} ng/mL^{∗}h | Cmax ^{∗} (ng/mL) | AUC day10-50h ^{∗}ng/mL^{*}h |
|---|---|---|---|
| Observed IR 45mg BID (6.5 hr between morning and afternoon dose) | 9556 | 476 | |
| Simulated IR 45mg BID (6.5 hr between morning and afternoon dose) | 9178 | 417 | 10323 |
| Simulated IR 45mg BID (7 hr between morning and afternoon dose) | 9092 | 414 | 10301 |
| Simulated IR 67.5mg BID (7 hr between morning and afternoon dose) | | 611 | 15057 |
| Simulated IR 90mg BID (7 hr between morning and afternoon dose) | | 818 | 20166 |
| Simulated IR 112.5mg BID (7 hr between morning and afternoon dose) | | 1025 | 25280 |
| Simulated IR 157.5mg BID (6.5 hr between morning and afternoon dose) | | 1450 | 41635 |
| MR-2 (once daily, 90mg) | 6780 | 258 | 7714 |
| MR-3 (once daily, **90mg**) | 4809 | 155 | 5647 |
| MR-1 (once daily, **90mg**) | 6890 | 269 | 7825 |
| MR-2 (once daily, **125mg**) | | 352 | 10427 |
| MR-3 (once daily, **125mg**) | | 209 | 7587 |
| MR-1 (once daily, **125mg**) | | 367 | 10583 |
| MR-2 (once daily, **135mg**) | | 381 | 11276 |
| MR-3 (once daily, **135mg**) | | 227 | 8207 |
| MR-1 (once daily, **135mg**) | | 397 | 11445 |
| MR-2 (once daily, **150mg**) | | 424 | 12551 |
| MR-3 (once daily, **150mg**) | | 252 | 9138 |
| MR-1 (once daily, **150mg**) | | 442 | 12737 |
| MR-2 (once daily, **180mg**) | | 510 | 15101 |
| MR-3 (once daily, **180mg**) | | 304 | 11001 |
| MR-1 (once daily, **180mg**) | | 531 | 15325 |
| MR-2 (once daily, **200mg**) | | 567 | 16802 |
| MR-3 (once daily, **200mg**) | | 338 | 12244 |
| MR-1 (once daily, **200mg**) | | 591 | 17050 |
| MR-2 (once daily, **225mg**) | | 639 | 18929 |
| MR-1 (once daily, **225mg**) | | 381 | 13864 |
| MR-3 (once daily, **225mg**) | | 666 | 19208 |
| MR-2 (once daily, **250mg**) | | 711 | 21057 |
| MR-3 (once daily, **250mg**) | | 424 | 15427 |
| MR-1 (once daily, **250mg**) | | 740 | 21367 |
| MR-2 (once daily, **315mg**) | | 897 | 26593 |
| MR-3 (once daily, **315mg**) | | 536 | 19493 |
| MR-1 (once daily, **315mg**) | | 934 | 26879 |

| | | | |
|---|---|---|---|
| ^{∗} AUC₀₋₃₈ and AUC₀₋₅₀ can be considered a good estimation of AUC_{inf} | | | |

**Table 7: Pridopidine Pharmacokinetic (PK) parameters following multiple daily doses of pridopidine (IR dosage form bid or MR dosage form QD), at steady state**

| **Dose and Regimen** | **Mean** | |
|---|---|---|
| | ***AUCtau,ss (hr*ng/mL)** | **Cmax,ss (ng/mL)** |
| Observed IR 45mg BID (6.5 hr between morning and afternoon dose) | 12547 | 807 |
| Simulated IR 45mg BID (6.5 hr between morning and afternoon dose) | 12634 | 675 |
| Simulated IR 45mg BID (7 hr between morning and afternoon dose) | 12641 | 670 |
| Simulated IR 67.5mg BID (6.5 hr between morning and afternoon dose) | 18951 | 1013 |
| Simulated IR 90mg BID (6.5 hr between morning and afternoon dose) | 25270 | 1351 |
| Simulated IR 112.5mg BID (6.5 hr between morning and afternoon dose) | 31585 | 1689 |
| Simulated IR 157.5mg BID (6.5 hr between morning and afternoon dose) | 43547 | 2336 |
| MR-2 (once daily, **90mg)** | 9479 | 495 |
| MR-3 (once daily, **90mg)** | 7236 | 340 |
| MR-1 (once daily, **90mg)** | 9591 | 508 |
| MR-2 (once daily, **100mg)** | 10532 | 550 |
| MR-3 (once daily, **100mg)** | 8052 | 378 |
| MR-1 (once daily, **100mg)** | 10657 | 564 |
| MR-2 (once daily, **125mg)** | 13165 | 688 |
| MR-3 (once daily, **125mg)** | 10051 | 472 |
| MR-1 (once daily, **125mg)** | 13322 | 706 |
| MR-2 (once daily, **135mg)** | 14218 | 743 |
| MR-3 (once daily, **135mg)** | 10855 | 510 |
| MR-1 (once daily, **135mg)** | 14387 | 762 |
| MR-2 (once daily, **150mg)** | 15798 | 826 |
| MR-3 (once daily, **150mg)** | 12061 | 567 |
| MR-1 (once daily, **150mg)** | 15986 | 847 |
| MR-2 (once daily, **180mg)** | 18957 | 991.1 |
| MR-3 (once daily, **180mg)** | 14474 | 680 |
| MR-1 (once daily, **180mg)** | 19183 | 1016 |
| MR-2 (once daily, **225mg)** | 23698 | 1239 |
| MR-1 (once daily, **225mg)** | 23981 | 1271 |
| MR-2 (once daily, **315mg)** | 32431 | 1712 |
| MR-3 (once daily, **315mg)** | 28068 | 1169 |
| MR-1 (once daily, **315mg)** | 32824 | 1757 |

| | | |
|---|---|---|
| ∗dosing interval (tau) = 24 hours. | | |

Dissolution data presented for the 90 mg dosage forms were experimentally tested as described in Examples 2-4. The dissolution data presented for dosage forms higher than 90 mg are presented based on a simulation which used the profiles of 90mg samples.

### Results and discussion of Examples 5-6:

Safety issues such as gastrointestinal disorders, psychiatric disorders, and cardiac adverse events are dose-dependent. Particularly for QT, these safety concerns are linked to maximum drug concentrations (Cₘₐₓ) rather than to AUC. However, it is not known if Cₘₐₓ or AUC are responsible for other adverse events such as CNS related and GI related adverse events.

The dosage forms of the present invention were shown to provide reduced maximal blood concentration (Cₘₐₓ) compared to b.i.d. adminitration of the same dose of drug per day, while maintaining AUC similar to those in previous studies (Huntington Study Group HART Investigators 2013, Yebenes 2011).

The calculated Cₘₐₓ resulting from the administration of 90mg Pridopidine in a MR dosage form of the present invention was found to be lower compared to Cₘₐₓ resulting from the 45mg IR admimistered b.i.d (Table 7), presenting a better safety profile. In addition the calculated AUC_{tau,ss} for the 90mg MR administration was comparable to AUC_{tau,ss} found in subjects administered with 45mg IR b.i.d in the MAD study. Similarly, the calculated Cₘₐₓ resulting from the administration of 135mg Pridopidine in a MR dosage form was lower compared to Cₘₐₓ resulting from the 67.5mg IR admimistered b.i.d; the calculated Cₘₐₓ resulting from the administration of 180mg Pridopidine in a MR dosage form was lower compared to Cₘₐₓ resulting from the 90mg IR admimistered b.i.d; the calculated Cₘₐₓ resulting from the administration of 225mg Pridopidine in a MR dosage form was lower compared to Cₘₐₓ resulting from the 112.5mg IR admimistered b.i.d, and the calculated Cₘₐₓ resulting from the administration of 315 Pridopidine in a MR dosage form was lower compared to Cₘₐₓ resulting from the 157.5mg IR admimistered b.i.d (Table 7). The AUC_{tau,ss} of these doses is higher than the AUC_{tau,ss} related to 45mg IR b.i.d. The AUC_{tau,ss} of these doses would be appreciated by the person skilled in the art to be relevant to therapeutically effective amounts of the formulation.

In addition, the calculated C_{max.ss} resulting from administration of MR dosage forms comprising 100mg and 125mg Pridopidine, was lower than the Cₘₐₓ resulting from 45mg IR admimistered b.i.d (a total dose of 90mg per day;see Table 7). For the 100mg MR dosage form, calculated AUC_{tau,ss} was about 80% of the 45mg IR b.i.d., and the AUC_{tau,ss} calculated for the 125mg MR dosage form was similar to 45mg IR b.i.d. Importantly, both were higher than mean AUC_{tau,ss} resulting from 45mg IR b.i.d. administration in the MAD study. These findings show that even for MR dosage forms comprising more than the same dose per day administed b.i.d., the safety profile was improved, while clinical activity maintained.

### Example 7

Three dosage forms of pridopidine are prepared according to Examples 2 and 3, MR-1, MR-2 and MR-3. Periodic oral administration of MR-1, MR-2 or MR-3 to a human patient afflicted with Huntington's Disease shows that the frequency of adverse events decreases compared to the frequence of adverse events in Example 1.

### Example 8

Three dosage forms of pridopidine are prepared according to Examples 2-3, MR-1, MR-2 and MR-3, however the amount of pridopidine is 100 mg (113 mg pridopidine HCl) and each of the other components of MR-1, MR-2 and MR-3 are increased proportionally. Periodic oral administration of the dose forms to a human patient afflicted with Hungington's Disease shows that the Cₘₐₓ is equal to or less than previously tested safe doses.

### Example 9

Three dosage forms of pridopidine are prepared according to Examples 2-3, MR-1, MR-2 and MR-3, however the amount of pridopidine is 125 mg (141 mg pridopidine HCl) and each of the other components of MR-1, MR-2 and MR-3 are increased proportionally. Periodic oral administration of the dose forms to a human patient afflicted with Huntington's Disease shows that the Cₘₐₓ is equal to or less than previously tested safe doses.

### Example 10

Three dosage forms of pridopidine are prepared according to Examples 2-3, MR-1, MR-2 and MR-3, however the amount of pridopidine is 135 mg (153 mg pridopidine HCl) and each of the other components of MR-1, MR-2 and MR-3 are increased proportionally. Periodic oral administration of the dose forms to a human patient afflicted with Huntington's Disease shows that the Cₘₐₓ is equal to or less than previously tested safe doses.

### Example 11

Three dosage forms of pridopidine are prepared according to Examples 2-3, MR-1, MR-2 and MR-3, however the amount of pridopidine is 150 mg (170 mg pridopidine HCl) and each of the other components of MR-1, MR-2 and MR-3 are increased proportionally. Periodic oral administration of the dose forms to a human patient afflicted with Huntington's Disease shows that the Cₘₐₓ is equal to or less than previously tested safe doses.

### Example 12

Three dosage forms of pridopidine are prepared according to Examples 2-3, MR-1, MR-2 and MR-3, however the amount of pridopidine is 180 mg (203 mg pridopidine HCl) and each of the other components of MR-1, MR-2 and MR-3 are increased proportionally. Periodic oral administration of the dose forms to a human patient afflicted with Huntington's Disease shows that the Cₘₐₓ is equal to or less than previously tested safe doses.

### Example 13

Three dosage forms of pridopidine are prepared according to Examples 2-3, MR-1, MR-2 and MR-3, however the amount of pridopidine is 225 mg (254 mg pridopidine HCl) and each of the other components of MR-1, MR-2 and MR-3 are increased proportionally. Periodic oral administration of the dose forms to a human patient afflicted with Huntington's Disease shows that the Cₘₐₓ is equal to or less than previously tested safe doses.

To summarize, the inventors of the present invention managed to fomulate therapeutically effective dosage forms with an increased safety profile compared to b.i.d. administration of the same dose per day or less.

Additionally, treatments of acute and chronic neurological and neuropsychiatric diseases, such as Huntington's disease, have the problem of treatment compliance because the patient or caretaker may forget to administer the medication. Accordingly, the oral dosage forms of the present invention provide advantages over the formerly known (b.i.d.) oral dosages. The oral dosage forms of the present invention are adapted for administration once daily, providing reduced pill burden for patients who resist treatment, increasing convenience for patients and caregivers and leading to greater compliance and less burden on family members.

### Example 14: PK study in Beagle dogs following single dose administration of the MR-1, MR-2 and MR-3 formulations.

The pharmacokinetics of pridopidine in male Beagle dogs was tested following oral administration of an immediate release (IR) formulation and three modified release (MR) formulations. The dogs were divided to 4 groups: Group 1 received one administration of formulation MR-1, Group 2 received one administration of formulation MR-2 and Group 3 received one administration of formulation MR-3. Each formulation comprised 90mg of Pridopidine. Pridopidine plasma concentration was measured at several time-points at 0.5-36 hours after administration.

Group 4 received 45 mg Pridopidine in IR formulation twice with 3h interval. Pridopidine plasma concentration was measured at several time-points at 0.5-36 hours after first administration.

The study was done under fasting condition starting 12 h before administration, and lasting additional 7h post first administration.

The results are presented in Table 8.

**Table 8**

| **Pridopidine** | | | | | | | |
|---|---|---|---|---|---|---|---|
| **Group No.** | **t1/2 mean** | **Tmax mean** | **Cmax mean** | **AUC_{INF} mean** | **AUC₀₋₂₄ mean** | **Frel_cmax** | **Frel_AUC₀₋ 24** |
| ***1 (n*=*4) (MR-1)*** | 4.2 | 1.75 | 1135 | 6221.9 | 6336 | 0.56 | 0.70 |
| **2 *(n=8) (MR-2)*** | 3.7 | 2 | 1203 | 7153 | 7038 | 0.59 | 0.78 |
| **3 *(n=7) (MR-3)*** | 4.8 | 3.5 | 907 | 6846 | 6743 | 0.45 | 0.75 |
| **4 *(n=6)*** | 5.4 | 4.0 | 2031 | 9125 | 9004 | 1.00 | 1.00 |

### Example 15: additional analysis of PK parameters.

The concentration of Pridopidine in the plasma samples in Example 14 was determined using liquid chromatography-tandem mass spectrometry LC-MS/MS. In an additional analysis, samples containing higher concentration of an analyte than the upper limit of the quantification (ULOQ: 2000 ng/ml pridopidine) was re-analyzed after 10 times dilution.

Briefly, the blood samples were centrifuged (within maximum 60 minutes after collection) at 2500 g at 5°C for 15 minutes. The frozen plasma samples were stored in an ultra-freezer ( -70±10°C). In the plasma samples the concentration of pridopidine were determined liquid chromatography-tandem mass spectrometry LC-MS/MS. As described, the samples containing higher concentration of an analyte than the upper limit of the quantification (ULOQ: 2000 ng/ml pridopidine) were re-analyzed after 10 times dilution .

For each formulation from the individual data the mean and S.D. values were calculated for each time-point generating a mean plasma concentrations versus time curve.

The pharmacokinetic analysis was performed using validated Phoenix WinNonlin Version 6.3 software (Pharsight Corporation, USA). The individual and mean pharmacokinetic parameters were calculated using a non-compartmental method.

### Results:

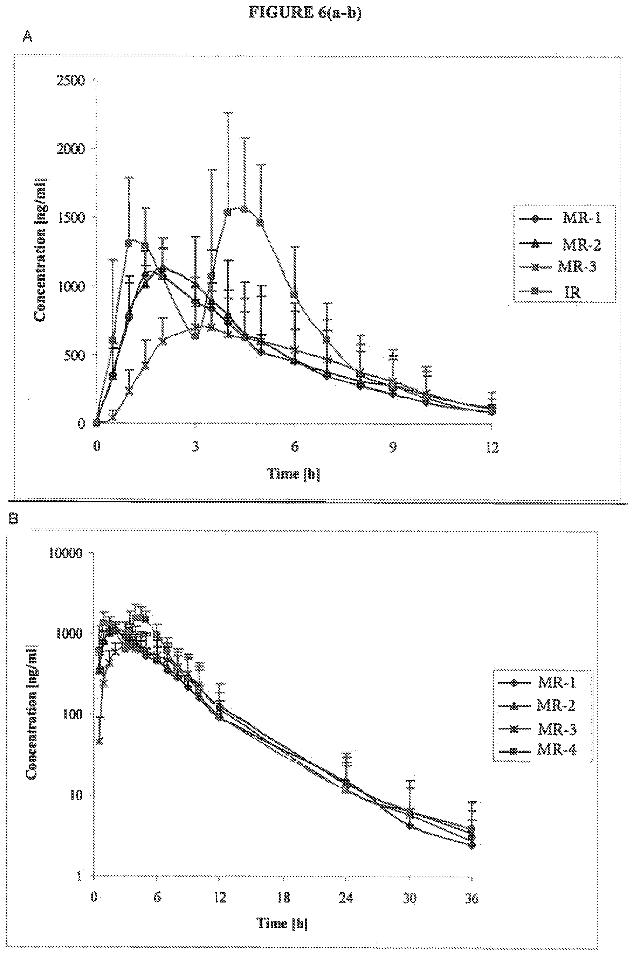

Figure 6 shows the mean plasma level curves (with S.D.) of pridopidine (6a-b) for formulations MR-1, MR-2, and MR-3. Two administrations of the immediate release (IR) formulation administered 3h apart resulted in an initial peak concentration followed by an initial decline then a second peak followed by the terminal elimination phase. In comparison, The MR formulation had a prolonged absorption from the MR formulations that resulted in a maximum concentration followed by a terminal elimination phase.

For all formulations the AUC_{(0-inf)} and Cₘₐₓ values were normalized to the nominal 12 mg/kg pridopidine dose. The T_{max,}dose normalized Cₘₐₓ and dose normalized total exposures (AUC_{(0-inf).nom}values) are summarized in Table 9.

**Table 9**

| | Group | | | | |
|---|---|---|---|---|---|
| | 1(MR-1) (S.D) | 2(MR-2) (S.D) | 3(MR-3) (S.D) | 4(IR) (1st dose) (S.D) | 4(IR) (2^{nd} dose) (S.D) |
| C_{max,norm} [ng/ml] | 1110 (200) | 1170 (235) | 803 (228) | 1550 (313) | 2030 (538) |
| Tₘₐₓ pridopidine | 1.94 (0.904) | 2.38 (1.03) | 3.63 (1.62) | 1.16 (0.351) | 3.91 (1.14) |
| AUC_{(0-inf),norm} [h∗ng/ml] | 6340 (1610) | 7010 (3520) | 6080 (2830) | | 9410 (3380) |

As can be seen, formulations MR-1 and MR-2 showed similar kinetic profiles while the most delayed absorption was observed for formulation MR-3. IR formulation resulted in the first pridopidine peak within the shortest period post-dose: at approximately 1 hour. For MR formulations the pridopidine peaks occurred later: at approximately 2 hours for formulation MR-1, 2.5 hours for formulation MR-2 and 3.5 hours for formulation MR-3.

The relative peak levels of the MR formulations compared to the higher, second peak level of the reference IR formulation (F_{rel_}Cₘₐₓ), and the relative total exposure (Fᵣₑₗ AUC_{inf}) were calculated from the total group means (Table 10).

**Table 10**

| | 1(MR-1) | 2 (MR-2) | 3 (MR-3) |
|---|---|---|---|
| Fᵣₑₗ Cₘₐₓ | **0.547** | **0.576** | **0.396** |
| Mean Fᵣₑₗ AUC_{inf} | 0.721 | 0.746 | 0.625 |

The results show that Cₘₐₓ resulting from the once daily administration of 90 mg Pridopidine in formulations MR-1, MR-2 and MR-3, was 55%, 58% and 40%, respectively, of the Cₘₐₓ resulting from 45 mg Pridopidine in IR formulation given bid. AUC_{inf} resulting from the single once daily administration of 90 mg Pridopidine in formulations MR-1, MR-2 and MR-3 was 72%, 75% and 63%, respectively of AUC_{inf} resulting from bid administration of 45 mg Pridopidine in the IR formulation.

### Example 16

Tablet dosage forms of pridopidine were prepared with granulates R1-R4 (Tables 3.1 or 3.2) and are presented in Table 11. The dissolution profile of these dosage forms are also listed in Table 11. Dissolution testing was performed using USP apparatus I at 100rpm, in 900mL purified water at 37°C. The detailed dissolution profiles of the dosage forms listed in Table 11 are shown in Table 12.

It would be appreciated by the person skilled in the art that the Dosage Forms presented in Table 11 have a modified release dosage form dissolution profile.

**Table 11**

| **No.** | **Use** | **MR-4** | **MR-5** | **MR-6** | **MR-7** | **MR-8** | **MR-9** | **MR-10** | **MR-11** |
|---|---|---|---|---|---|---|---|---|---|
| **Composition** | - | mg/Tab | mg/Tab | mg/Tab | mg/Tab | mg/Tab | mg/Tab | mg/Tab | mg/Tab |
| Granules¹ | - | **R1** 122.0 | **R4** 163.2 | R3 304.8 | R3 304.8 | **R1** 122.0 | **R1** 122.0 | R2 300.0 | **R4** 163.2 |
| Calcium Phosphate Dibasic | Insoluble filler | ^{∗} | ^{∗} | ^{∗} | ^{∗} | 154.0 | ^{∗} | ^{∗} | ^{∗} |
| Hydroxypropyl Methyl Cellulose (HPMC) Methocel K100 PR CR | Hydrophilic carrier | 122.0 | ^{∗} | 90.0 | 90.0 | 120.0 | 120.0 | 90.0 | 150.0 |
| (HPMC) Methocel K15M CR | Hydrophilic carrier | ^{∗} | ^{∗} | ^{∗} | ^{∗} | ^{∗} | ^{∗} | ^{∗} | 25.0 |
| Hydrogenated Castor Oil | Hydrophobic carrier | 30.0 | 175.0 | ^{∗} | 60.0 | ^{∗} | ^{∗} | ^{∗} | ^{∗} |
| Aerosil | Flow agent | ^{∗} | ^{∗} | 5.0 | 5.0 | 2.0 | 2.0 | 5.0 | * |
| Mg.Stearate | Lubricant | 2.0 | 1.8 | 5.2 | 5.2 | 2.0 | 2.0 | 5.0 | 1.8 |
| LubriTose Blue² | Lubricant | ^{∗} | 160.0 | ^{∗} | ^{∗} | ^{∗} | ^{∗} | ^{∗} | ^{∗} |
| LubriTose Yellow | Lubricant | ^{∗} | ^{∗} | ^{∗} | ^{∗} | ^{∗} | ^{∗} | ^{∗} | 160.0 |
| Lactose Anhydrous | Soluble filler | ^{∗} | ^{∗} | 150.0 | 75.0 | ^{∗} | 154.0 | 100 | ^{∗} |
| Tablet Weight | | 276.0 | 500.0 | 555.0 | 540.0 | 400.0 | 400.0 | 500.0 | 500.0 |
| Dissolution profile³ | | 12h release | **9-10h release** | **9h release** | **9h release** | 9h **release** | 9h **release** | **9h release** | **9-12 release** |

| | | | | | | | | | |
|---|---|---|---|---|---|---|---|---|---|
| ¹Granules R1, R2, R3, **and** R4 are listed in Table 3.1, or 3.2. ² Lactose + (2%-10% Glyceryl MonoStearate): yellow contain 10% GMS and blue contain 2% GMS. ³ Dissolution testing was performed using USP, apparatus I at 100rpm, in 900mL purified water at 37°C. | | | | | | | | | |

**Table 12: Dissolution Profiles of the Formulations in Table 11**

| | **Time/ Batch** | **5min** | **1h** | **3h** | **6h** | **9h** | **12h** |
|---|---|---|---|---|---|---|---|
| Release Rate (%) | **MR-4** | 8 | 37 | 62 | 83 | 94 | 99 |
| | **MR-5** | 12 | 40 | 65 | 83 | 94 | 101 |
| | **MR-6** | 3 | 30 | 60 | 84 | 92 | 95 |
| | **MR-7** | 5 | 36 | 67 | 88 | 97 | 100 |
| | **MR-8** | 6 | 37 | 68 | 91 | 103 | |
| | **MR-9** | 3 | 32 | 68 | 94 | 105 | |
| | **MR-10** | 4 | 38 | 72 | 95 | 102 | |
| | **MR-11** | 5 | 32 | 60 | 82 | 93 | 100 |

### References:

Clinicaltrials.gov Clinical Trial Identifier NCT02006472, "A Phase 2, to Evaluating the Safety and Efficacy of Pridopidine Versus Placebo for Symptomatic Treatment in Patients With Huntington's Disease."
de Yebenes JG, Landwehrmeyer B, Squitieri F, Reilmann R, Rosser A, Barker RA, Saft C, Magnet MK, Sword A, Rembratt A, Tedroff J; MermaiHD study investigators, "Pridopidine for the treatment of motor function in patients with Huntington's disease (MermaiHD): a phase 3, randomised, double-blind, placebo-controlled trial," Lancet Neurol. 2011 Dec;10(12):1049-57. doi: 10.1016/S1474-4422(11)70233-2. Epub 2011 Nov 7.
Huntington Study Group HART Investigators, "A randomized, double-blind, placebo-controlled trial of pridopidine in Huntington's disease," Mov Disord. 2013 Sep;28(10):1407-15. doi: 10.1002/mds.25362. Epub 2013 Feb 28.
Helldén A, Panagiotidis G, Johansson P, Waters N, Waters S, Tedroff J, Bertilsson L. "The dopaminergic stabilizer pridopidine is to a major extent N-depropylated by CYP2D6 in humans" Eur J Clin Pharmacol. 2012 Sep; 68(9):1281-6. Epub 2012 Mar 8.
Lindskov Krog P, Osterberg O, Gundorf Drewes P, Rembratt Å, Schultz A, Timmer W. "Pharmacokinetic and tolerability profile of pridopidine in healthy-volunteer poor and extensive CYP2D6 metabolizers, following single and multiple dosing" Eur J Drug Metab Pharmacokinet. 2013 Mar;38(1):43-51. Epub 2012 Sep 5.
Østerberg, et al. "A single center, randomized, placebo-controlled, double-blind study to evaluate the safety, tolerability, and pharmacokinetics of multiple-ascending doses of pridopidine in healthy volunteers" Poster presented at Sixth Annual Huntington Disease Clinical Research Symposium, Nov 2012, Seattle, Washington, USA. Neurotherapeutics

## Claims

1. A modified release solid oral dosage form comprising a therapeutically effective amount of Pridopidine or a pharmaceutically acceptable salt thereof, and at least one pharmaceutically acceptable rate controlling excipient, wherein the rate controlling excipient is a hydrophilic or hydrophobic polymer selected from hydrogenated castor oil, polyethylene oxide, ethyl cellulose, hydroxypropyl methylcellulose (HPMC), hydroxypropyl cellulose (HPC), polyvinyl alcohol (PVA), vinyl alcohol polymer, polyacrylates, polymethacrylates, ethyl acrylate-methyl methacrylate copolymers, glyceryl monostearate, and mixtures thereof, wherein the solid oral dosage form provides an *in vivo* plasma pridopidine concentration profile having a Mean Cₘₐₓ of 1,400 ng/ml or less or having a Cₘₐₓ from 244 ng/ml to 1568 ng/ml at steady state.

2. The modified release solid oral dosage form according to claim 1, wherein the rate controlling excipient is a combination of at least a hydroxypropyl methylcellulose (HPMC) and hydrogenated castor oil.

3. The modified release solid oral dosage form according to claim 1, wherein the rate controlling excipient comprises hydroxypropyl methylcellulose (HPMC) or hydrogenated castor oil.

4. The modified release solid oral dosage form according to claim 1, wherein the solid oral dosage form provides an *in vivo* plasma pridopidine concentration profile having a Cₘₐₓ from 244 ng/ml to 1002 ng/ml when given as single dose.

5. The modified release solid oral dosage form of any of claims 1-4, wherein the solid oral dosage form provides an *in vivo* plasma pridopidine concentration profile having a Mean Cₘₐₓ and Mean AUCₜₐᵤ which is lower than a Mean Cₘₐₓ and Mean AUCₜₐᵤ respectively resulting from the b.i.d. administration of an immediate release solid oral dosage form which contains half the amount of the pridopidine or a pharmaceutically acceptable salt thereof.

6. The modified release solid oral dosage form of any one of claims 1-5, wherein the mean time required to reach the maximal plasma, serum or blood concentration of the drug, following administration of the drug is more than 2 hours.

7. The modified release solid oral dosage form according to any one of claims 1-6, wherein the total amount of the rate controlling excipients is from 8% to 70% of the total weight of the dosage form, from 10% to 50% of the total weight of the dosage form, or from 20% to 50% of the total weight of the dosage form, from 30% to 50% or from 30% to 40% of the total weight of the dosage form.

8. The modified release solid oral dosage form of any one of claims 1-7, wherein the dosage form comprises between 22.5mg to 350mg pridopidine or pharmaceutically acceptable salt thereof.

9. The modified release solid oral dosage form of any one of claims 1-8, wherein the pridopidine or pharmaceutically acceptable salt thereof comprises from 15% to 60% by weight of the dosage form.

10. The modified release solid oral dosage form according to any one of claims 1-9 wherein the weight ratio of pridopidine or pharmaceutically acceptable salt thereof to the rate controlling excipient is from 0.2:1 to 1:1, preferably from 0.3:1 to 0.8:1, more preferably 0.5:1 to 0.7:1.

11. The modified release solid oral dosage form of any one of claims 1-10, further comprising ethylcellulose, wherein the total amount of the ethylcellulose is from 0.5% to 10% of the total weight of the dosage form.

12. The modified release solid oral dosage form of any one of claims 1-11, further comprising a mucoadhesive, wherein the mucoadhesive is selected from water soluble or water insoluble hydrophilic polymers, polymers that have swellable networks, hydrogels, and polymers with groups that can cross-link with other polymers or with a mucous membrane, wherein preferably the mucoadhesive is polyethylene oxide.

13. The modified release solid oral dosage form according to any one of claims 1-12, wherein the pharmaceutically acceptable salt of pridopidine is hydrochloride salt.

14. The modified release solid oral dosage form according to any one of claims 1-13, wherein the oral dosage form comprises capsules, tablets, pellets, granules, powders coated or uncoated and combination thereof.

15. A pharmaceutical formulation comprising the modified release solid oral dosage form of any one of claims 1-14, and one or more pharmaceutically acceptable carriers or excipients selected from binders, fillers, plasticizers, glidants and lubricants and mixtures thereof.

16. The pharmaceutical composition of claim 15, wherein:
(a) the binder is starch, pregeletinized starch, polyethylene oxide, cellulose polymers, hydroxypropylmethyl cellulose, hydroxypropylcellulose, methylcellulose, hydroxyethyl cellulose, polyvinylpyrrolidone, polyvinyl alcohol or a mixture thereof;
(b) the filler is microcrystalline cellulose, sugar spheres, lactose, sorbitol, dextrose, sucrose, mannitol, dibasic or tribasic calcium phosphate, calcium sulfate, starch, retalac or a mixture thereof, optionally wherein the filler is a silicified microcrystalline cellulose;
(c) the plasticizer is polyethylene glycol, triethyl citrate, tributyl citrate, glycerin, dibutyl sebacate, triacetin, diethylphthalat or a mixture thereof;
(d) the glidant is starch, pregelatinized starch, silicone dioxide, colloidal silicone dioxide, talc or a mixture thereof;
(e) the lubricant is sodium stearyl fumarate, stearic acid, magnesium stearate, calcium stearate, zinc stearate, talc, glyceryl behenate, glyceryl monostearate, or a mixture thereof.

17. A modified release solid oral dosage form or pharmaceutical formulation according to any one of claims 1-14, or a pharmaceutical composition of any of claims 15 or 16, for use in the treatment of Huntington's Disease, Parkinson's disease, iatrogenic and non-iatrogenic Parkinsonism, dyskinesias, dystonias, Tourette's disease, iatrogenic and non-iatrogenic psychoses and hallucinoses, schizophrenia disorder or schizophreniform disorder, mood and anxiety disorders, manodepressive illness, depression, obsessive-compulsive disease, a sleep disorder, autism spectrum disorder, ADHD, age-related cognitive impairment, abuse of alcohol and substances used as narcotics, Alzheimer's disease or Retts syndrome, wherein the modified release solid oral dosage form or pharmaceutical formulation is adapted for once daily administration.

## Patentansprüche

1. Feste orale Dosierungsform mit modifizierter Freisetzung, umfassend eine therapeutisch wirksame Menge Pridopidin oder ein pharmazeutisch annehmbares Salz davon und mindestens einen pharmazeutisch annehmbaren die Freisetzungsrate steuernden Hilfsstoff, wobei der die Freisetzungsrate steuernde Hilfsstoff ein hydrophiles oder hydrophobes Polymer ist, das aus hydriertem Rizinusöl, Polyethylenoxid, Ethylcellulose, Hydroxypropylmethylcellulose (HPMC), Hydroxypropylcellulose (HPC), Polyvinylalkohol (PVA), Vinylalkoholpolymer, Polyacrylaten, Polymethacrylaten, Ethylacrylat-Methylmethacrylat-Copolymeren, Glycerylmonostearat und Gemischen davon ausgewählt ist, wobei die feste orale Dosierungsform ein In-Vivo-Konzentrationsprofil von Pridopidin in Plasma von Mean Cₘₐₓ von 1,400 ng/ml oder darunter oder von Cₘₐₓ zwischen 244 ng/ml und 1568 ng/ml im Steady State bereitstellt.

2. Feste orale Dosierungsform mit modifizierter Freisetzung nach Anspruch 1, wobei der die Freisetzungsrate steuernde Hilfsstoff eine Kombination aus mindestens einer Hydroxypropylmethylcellulose (HPMC) und hydriertem Rizinusöl ist.

3. Feste orale Dosierungsform mit modifizierter Freisetzung nach Anspruch 1, wobei der die Freisetzungsrate steuernde Hilfsstoff Hydroxypropylmethylcellulose (HPMC) oder hydriertes Rizinusöl umfasst.

4. Feste orale Dosierungsform mit modifizierter Freisetzung nach Anspruch 1, wobei die feste orale Dosierungsform bei Gabe als Einzeldosis ein In-Vivo-Konzentrationsprofil von Pridopidin in Plasma von Cₘₐₓ zwischen 244 ng/ml und 1002 ng/ml bereitstellt.

5. Feste orale Dosierungsform mit modifizierter Freisetzung nach einem der Ansprüche 1-4, wobei die feste orale Dosierungsform ein In-Vivo-Konzentrationsprofil von Pridopidin in Plasma von Mean Cₘₐₓ und Mean AUCₜₐᵤ bereitstellt, das niedriger ist als jeweils ein Mean Cₘₐₓ und Mean AUCₜₐᵤ, die sich bei der zweimal täglichen Verabreichung einer festen oralen Dosierungsform mit Sofortfreisetzung ergeben, welche die Hälfte der Menge des Pridopidin oder eines pharmazeutisch annehmbaren Salzes davon beinhaltet.

6. Feste orale Dosierungsform mit modifizierter Freisetzung nach einem der Ansprüche 1-5, wobei die mittlere Zeit, die nach der Verabreichung des Arzneimittels erforderlich ist, um den Maximalwert der Konzentration des Arzneimittels in Plasma, Serum oder Blut zu erreichen, mehr als 2 Stunden beträgt.

7. Feste orale Dosierungsform mit modifizierter Freisetzung nach einem der Ansprüche 1-6, wobei die Gesamtmenge des die Freisetzungsrate steuernden Hilfsmittels zwischen 8 % und 70 % des Gesamtgewichts der Dosierungsform, zwischen 10 % und 50 % des Gesamtgewichts der Dosierungsform oder zwischen 20 % und 50 % des Gesamtgewichts der Dosierungsform, zwischen 30 % und 50 % oder zwischen 30 % und 40 % des Gesamtgewichts der Dosierungsform beträgt.

8. Feste orale Dosierungsform mit modifizierter Freisetzung nach einem der Ansprüche 1-7, wobei die Dosierungsform zwischen 22,5 mg und 350 mg Pridopidin oder eines pharmazeutisch annehmbaren Salzes davon umfasst.

9. Feste orale Dosierungsform mit modifizierter Freisetzung nach einem der Ansprüche 1-8, wobei das Pridopidin oder das pharmazeutisch annehmbare Salz davon zwischen 15 Gew-% und 60 Gew-% der Dosierungsform umfasst.

10. Feste orale Dosierungsform mit modifizierter Freisetzung nach einem der Ansprüche 1-9, wobei das Gewichtsverhältnis von Pridopidin oder des pharmazeutisch annehmbaren Salzes davon zu dem die Freisetzungsrate steuernden Hilfsstoff zwischen 0,2:1 und 1:1, bevorzugt zwischen 0,3:1 bis 0,8:1, noch bevorzugter zwischen 0,5:1 und 0,7:1 liegt.

11. Feste orale Dosierungsform mit modifizierter Freisetzung nach einem der Ansprüche 1-10, ferner umfassend Ethylcellulose, wobei die Gesamtmenge der Ethylcellulose zwischen 0,5 % und 10 % des Gesamtgewichts der Dosierungsform beträgt.

12. Feste orale Dosierungsform mit modifizierter Freisetzung nach einem der Ansprüche 1-11, ferner umfassend ein Mucoadhäsiv, wobei das Mucoadhäsiv aus wasserlöslichen oder wasserunlöslichen hydrophilen Polymeren, Polymeren mit schwellfähigen Netzwerken, Hydrogelen und Polymeren mit Gruppen, die mit anderen Polymeren oder mit einer Schleimhaut vernetzungsfähig sind, ausgewählt sind, wobei das Mucoadhäsiv bevorzugt Polyethylenoxid ist.

13. Feste orale Dosierungsform mit modifizierter Freisetzung nach einem der Ansprüche 1-12, wobei das pharmazeutisch annehmbare Salz von Pridopidin Hydrochlorid-Salz ist.

14. Feste orale Dosierungsform mit modifizierter Freisetzung nach einem der Ansprüche 1-13, wobei die orale Dosierungsform jeweils beschichtete oder unbeschichtete Kapseln, Tabletten, Pellets, Granulate, Pulver und Kombinationen davon umfasst.

15. Pharmazeutische Formulierung, umfassend feste orale Dosierungsform mit modifizierter Freisetzung nach einem der Ansprüche 1-14 und ein oder mehr pharmazeutisch annehmbare(r) Träger oder Hilfsstoff(e), die aus Bindern, Füllern, Weichmachern, Gleit- und Schmiermitteln sowie Gemischen davon ausgewählt ist (sind).

16. Pharmazeutische Zusammensetzung nach Anspruch 15, wobei Folgendes gilt:
(a) der Binder ist eine Stärke, vorgelatinierte Stärke, Polyethylenoxid, Cellulose-Polymere, Hydroxypropylmethylcellulose, Hydroxypropylcellulose, Methylcellulose, Hydroxyethylcellulose, Polyvinylpyrrolidon, Polyvinylalkohol oder ein Gemisch davon;
(b) der Füller ist Mikrokristalline Cellulose, Zuckerkugeln, Lactose, Sorbitol, Dextrose, Sucrose, Mannitol, dibasisches oder tribasisches Calciumphosphat, Calciumsulfat, Stärke, Retalac oder ein Gemisch davon, wobei der Füller wahlweise verkieselte Mikrokristalline Cellulose ist;
(c) der Weichmacher ist Polyethylenglycol, Triethylcitrat, Tributylcitrate, Glycerin, Dibutylsebacat, Triacetin, Diethylphthalat oder ein Gemisch davon;
(d) das Gleitmittel ist Stärke, vorgelatinisierte Stärke, Siliciumdioxid, kolloidales Siliziumdioxid, Talkum oder ein Gemisch davon ist;
(e) das Schmiermittel ist Natriumstearylfumarat, Stearinsäure, Magnesiumstearat, Calciumstearat, Zinkstearat, Talkum, Glycerylbehenat, Glycerylmonostearat oder ein Gemisch davon.

17. Feste orale Dosierungsform mit modifizierter Freisetzung oder pharmazeutische Formulierung nach einem der Ansprüche 1-14 oder eine pharmazeutische Zusammensetzung nach einem der Ansprüche 15 oder 16, zur Anwendung bei der Behandlung von Chorea Huntington, Morbus Parkinson, iatrogenem und nicht-iatrogenem Parkinsonismus, Dyskinesien, Dystonien, Tourette-Syndrom, iatrogenen und nichtiatrogenen Psychosen und Halluzinosen, schizophrenen Störungen oder Schizophreniformen Störungen, Stimmungs- und Angststörungen, manisch-depressiven Erkrankungen, Depression, zwangsneurotischen Erkrankungen, Schlafstörungen, Störungen auf dem Autismus-Spektrum, ADHD, altersbedingten kognitiven Beeinträchtigungen, Alkohol- und anderem Substanzmissbrauch, wie etwa Narkotika-Missbrauch, Morbus Alzheimer oder Retts-Syndrom, wobei die feste orale Dosierungsform mit modifizierter Freisetzung oder die pharmazeutische Formulierung für eine einmal tägliche Verabreichung adaptiert ist.

## Revendications

1. Forme posologique orale solide à libération modifiée comprenant une quantité thérapeutiquement efficace de Pridopidine ou d'un sel pharmaceutiquement acceptable de celle-ci, et au moins un excipient à libération contrôlée pharmaceutiquement acceptable, dans laquelle l'excipient à libération contrôlée est un polymère hydrophile ou hydrophobe choisi parmi l'huile de ricin hydrogénée, l'oxyde de polyéthylène, l'éthylcellulose, l'hydroxypropylméthylcellulose (HPMC), l'hydroxypropylcellulose (HPC), l'alcool polyvinylique (PVA), le polymère d'alcool vinylique, les polyacrylates, les polyméthacrylates, les copolymères d'acrylate d'éthyle et de méthacrylate de méthyle, le monostéarate de glycéryle et leurs mélanges, dans laquelle la forme posologique orale solide fournit un profil de concentration plasmatique de pridopidine *in vivo* ayant une Cₘₐₓ moyenne de 1 400 ng/ml ou moins ou ayant une Cₘₐₓ de 244 ng/ml à 1 568 ng/ml à l'état d'équilibre.

2. Forme posologique orale solide à libération modifiée selon la revendication 1, dans laquelle l'excipient à libération contrôlée est une combinaison d'au moins une hydroxypropylméthylcellulose (HPMC) et d'huile de ricin hydrogénée.

3. Forme posologique orale solide à libération modifiée selon la revendication 1, dans laquelle l'excipient à libération contrôlée comprend de l'hydroxypropylméthylcellulose (HPMC) ou de l'huile de ricin hydrogénée.

4. Forme posologique orale solide à libération modifiée selon la revendication 1, dans laquelle la forme posologique orale solide fournit un profil de concentration plasmatique de pridopidine *in vivo* ayant une Cmax de 244 ng/ml à 1002 ng/ml lorsqu'elle est administrée en dose unique.

5. Forme posologique orale solide à libération modifiée selon l'une quelconque des revendications 1 à 4, dans laquelle la forme posologique orale solide fournit un profil de concentration plasmatique de pridopidine *in vivo* ayant une C-max moyenne et un AUCₜₐᵤ moyen qui est inférieur à une C-max moyenne et un AUCₜₐᵤ moyen résultant respectivement de l'administration BID d'une forme posologique orale solide à libération immédiate qui contient la moitié de la quantité de pridopidine ou d'un sel pharmaceutiquement acceptable de celle-ci.

6. Forme posologique orale solide à libération modifiée selon l'une quelconque des revendications 1 à 5, dans laquelle le temps moyen requis pour atteindre la concentration plasmatique, sérique ou sanguine maximale du médicament après administration du médicament est supérieur à 2 heures.

7. Forme posologique orale solide à libération modifiée selon l'une quelconque des revendications 1 à 6, dans laquelle la quantité totale des excipients à libération contrôlée est de 8 % à 70 % du poids total de la forme posologique, de 10 % à 50 % du poids total de la forme posologique, ou de 20 % à 50 % du poids total de la forme posologique, de 30 % à 50 % ou de 30 % à 40 % du poids total de la forme posologique.

8. Forme posologique orale solide à libération modifiée selon l'une quelconque des revendications 1 à 7, dans laquelle la forme posologique comprend entre 22,5 mg et 350 mg de pridopidine ou d'un sel pharmaceutiquement acceptable de celle-ci.

9. Forme posologique orale solide à libération modifiée selon l'une quelconque des revendications 1 à 8, dans laquelle la pridopidine ou un sel pharmaceutiquement acceptable de celle-ci constitue de 15 % à 60 % en poids de la forme posologique.

10. Forme posologique orale solide à libération modifiée selon l'une quelconque des revendications 1 à 9, dans laquelle le rapport pondéral de la pridopidine ou de son sel pharmaceutiquement acceptable à l'excipient à libération contrôlée est de 0,2:1 à 1:1, de préférence de 0,3:1 à 0,8:1, plus préférablement 0,5:1 à 0,7:1.

11. Forme posologique orale solide à libération modifiée selon l'une quelconque des revendications 1 à 10, comprenant en outre de l'éthylcellulose, dans laquelle la quantité totale d'éthylcellulose est de 0,5 % à 10 % du poids total de la forme posologique.

12. Forme posologique orale solide à libération modifiée selon l'une quelconque des revendications 1 à 11, comprenant en outre un mucoadhésif, dans laquelle le mucoadhésif est choisi parmi des polymères hydrophiles hydrosolubles ou insolubles dans l'eau, des polymères qui ont des réseaux gonflables, des hydrogels et des polymères avec des groupes qui peuvent réticuler avec d'autres polymères ou avec une membrane muqueuse, dans laquelle le mucoadhésif est de préférence l'oxyde de polyéthylène.

13. Forme posologique orale solide à libération modifiée selon l'une quelconque des revendications 1 à 12, dans laquelle le sel pharmaceutiquement acceptable de pridopidine est le sel de chlorhydrate.

14. Forme posologique orale solide à libération modifiée selon l'une quelconque des revendications 1 à 13, dans laquelle la forme posologique orale comprend des capsules, des comprimés, des pastilles, des granules, des poudres enrobées ou non enrobées et une combinaison de ceux-ci.

15. Formulation pharmaceutique comprenant la forme posologique orale solide à libération modifiée selon l'une quelconque des revendications 1 à 14, et un ou plusieurs supports ou excipients pharmaceutiquement acceptables choisis parmi des liants, des charges, des plastifiants, des agents de glissement et des lubrifiants et leurs mélanges.

16. Composition pharmaceutique selon la revendication 15, dans laquelle :
(a) le liant est l'amidon, l'amidon prégélifié, l'oxyde de polyéthylène, les polymères de cellulose, l'hydroxypropylméthylcellulose, l'hydroxypropylcellulose, la méthylcellulose, l'hydroxyéthylcellulose, la polyvinylpyrrolidone, l'alcool polyvinylique ou un mélange de ceux-ci ;
(b) la charge est de la cellulose microcristalline, des sphères de sucre, du lactose, du sorbitol, du dextrose, du saccharose, du mannitol, du phosphate de calcium dibasique ou tribasique, du sulfate de calcium, de l'amidon, du retalac ou un mélange de ceux-ci, la charge étant éventuellement une cellulose microcristalline silicifiée ;
(c) le plastifiant est le polyéthylène glycol, le citrate de triéthyle, le citrate de tributyle, la glycérine, le sébaçate de dibutyle, la triacétine, le phtalate de diéthyle ou un mélange de ceux-ci ;
(d) l'agent de glissement est l'amidon, l'amidon prégélatinisé, le dioxyde de silicone, le dioxyde de silicone colloïdal, le talc ou un mélange de ceux-ci ;
(e) le lubrifiant est le fumarate de stéaryle de sodium, l'acide stéarique, le stéarate de magnésium, le stéarate de calcium, le stéarate de zinc, le talc, le béhénate de glycéryle, le monostéarate de glycéryle, ou un mélange de ceux-ci.

17. Forme posologique orale solide à libération modifiée ou formulation pharmaceutique selon l'une quelconque des revendications 1 à 14, ou composition pharmaceutique selon l'une quelconque des revendications 15 ou 16, pour une utilisation dans le traitement de la maladie de Huntington, de la maladie de Parkinson, du parkinsonisme iatrogène et non iatrogène, des dyskinésies, des dystonies, de la maladie de Gilles de la Tourette, des psychoses et hallucinoses iatrogènes et non iatrogènes, de la schizophrénie ou d'un trouble schizophréniforme, des troubles de l'humeur et de l'anxiété, des maladies manodépressives, de la dépression, de la maladie obsessionnelle-compulsive, des troubles du sommeil, d'un trouble du spectre autistique, du TDAH, des troubles cognitifs liés à l'âge, de l'abus d'alcool et de substances utilisées comme narcotiques, de la maladie d'Alzheimer ou du syndrome de Retts, la forme posologique orale solide à libération modifiée ou la formulation pharmaceutique étant adaptée pour une administration quotidienne unique.
